# EUROPEAN PATENT APPLICATION

(11) **EP 3 970 798 A1**
(43) Date of publication of application: **23.03.2022**
(21) Application number: 20197031.6
(22) Date of filing: 18.09.2020
(51) Int. Cl.: A61P 31/14, C07K 16/10

(54) **SARS-COV-2-NANOBODIES**

(71) Applicant: NMI Naturwissenschaftliches und Medizinisches Institut an der Universität Tübingen, 72770 Reutlingen (DE)
(72) Inventor: Rothbauer, Ulrich, 72072 Tübingen (DE); Wagner, Teresa, 72393 Burladingen (DE); Kaiser, Philipp, 72076 Tübingen (DE); Schneiderhan, Nicole, 72827 Wannweil (DE)
(74) Representative: Witte, Weller & Partner Patentanwälte mbB

(57) **Abstract**

The present invention relates to novel nanobodies directed against SARS-CoV-2, as well as to combination of the novel nanobodies, as well as to their use in therapy, diagnosis and prognosis.

## Description

The present invention relates to nanobodies against SARS-CoV-2 (Severe acute respiratory syndrome coronavirus type 2), which bind to the Receptor binding domain (RBD) of SARS-CoV-2, as well as to the use of such nanobodies, in particular prophylactic, therapeutic or diagnostic purposes, such as the prophylactic, therapeutic or diagnostic purposes regarding SARS-CoV-2 /SARS-CoV-2-infections.

The present invention also relates to polypeptides comprising or essentially consisting of one or more of such nanobodies. The invention also relates to nucleic acids encoding such nanobodies and polypeptides, to compositions comprising such nanobodies or polypeptides, and to uses of such polypeptides, nucleic acids, and compositions, in particular for prophylactic, therapeutic or diagnostic purposes, such as the prophylactic, therapeutic or diagnostic purposes regarding SARS-CoV-2.

### BACKGROUND

Severe acute respiratory syndrome coronavirus 2 (SARS-CoV-2) is the strain of coronavirus that causes coronavirus disease 2019 (COVID-19), the respiratory illness responsible for the COVID-19 pandemic. The virus, also colloquially known as simply the coronavirus (because of its crown-like appearance under an electron microscope due to the presence of spike glycoproteins on the envelope) is a positive-sense single-stranded RNA virus that is highly contagious in humans. It primarily spreads between people through close contact and via respiratory droplets produced from coughs or sneezes. Per mid-September 2020, the SARS-CoV-2-pandemic has caused the death of more than 850,000 people world-wide, and many countries suffer from severe lock-downs and dramatic economic costs.

As per today, there is no specific antiviral treatment recommended for COVID-19, and no vaccine is currently available. The treatment is symptomatic, and oxygen therapy represents the first step for addressing respiratory impairment. Non-invasive (NIV) and invasive mechanical ventilation (IMV) may be necessary in cases of respiratory failure refractory to oxygen therapy.

Also, among other therapeutic strategies, systemic corticosteroids have been used, such as dexamethasone. Regarding antiviral treatments, the use of remdesivir - an inhibitor of RNA polymerase - has been proposed since preclinical studies suggested that it could be effective for both prophylaxis and therapy of SARS-CoV-2-infections; also, alpha-interferon has been proposed, as well as several anti-flu drugs such as oseltamivir, favipiravir, and arbidol. As immunomodulatory therapy, chloroquine and hydroxychloroquine have been proposed, however, due to their severe side effects, further studies are needed for recommending their use in Covid-19 therapy.

Antibodies taken from the blood of healed individuals represent a therapeutic option currently under study. It is calculated that the dose of antibodies necessary for the treatment of a single patient with SARS-CoV-2, requires the removal of antibodies carried out by at least three patients recovered from the SARS-CoV-2 infection.

Neutralizing antibodies (nAbs) targeting the causative agent of the disease, the severe acute respiratory syndrome coronavirus 2 (SARS-CoV-2), have gained substantial interest for prophylactic and therapeutic options and could help guide vaccine design. However, no clinically approved antibody has been provided so far, and there still is the high need for effective vaccination, diagnosis and therapeutic treatment.

Accordingly, it is an object of the invention to provide for novel neutralizing binding molecules as tools for acute treatment of COVID-19, as tools for monitoring the presence of a neutralizing immune response in infected or vaccinated individuals, and as diagnostic tools.

### SUMMARY

The present invention provides a nanobody directed against the SARS-CoV-2 (Severe acute respiratory syndrome coronavirus type 2) comprising:
a) the amino acid sequences (i) VAYGNMLRGYVVG (SEQ ID NO: 1) as CDR1, (ii) IDTSGEKKK (SEQ ID NO: 2) as CDR2 and (iii) NADAPWPPRPYSVIGTRTG (SEQ ID NO: 3) as CDR3; or
b) the amino acid sequences (i) VGSGFLFSGYAMN (SEQ ID NO: 4) as CDR1, (ii) ISNAGDITH (SEQ ID NO: 5) as CDR2 and (iii) HAPGVRVASGERND (SEQ ID NO: 6) as CDR3; or
c) the amino acid sequences (i) VGSGFTFSGYAIN (SEQ ID NO: 7) as CDR1, (ii) ISNAGDLTH (SEQ ID NO: 8) as CDR2 and (iii) HAPGVRVGTGERND (SEQ ID NO: 9) as CDR3 ; or
d) the amino acid sequences (i) SASGFAFSSVSMS (SEQ ID NO: 10) as CDR1, (ii) IDRDGGNGN (SEQ ID NO: 11) as CDR2 and (iii) RLGTRDHIMS (SEQ ID NO: 12) as CDR3; or
e) the amino acid sequences (i) ETSRSSLDAYAIG (SEQ ID NO: 13) as CDR1, (ii) ISSSSMRTE (SEQ ID NO: 14) as CDR2 and (iii) AAAGEYGRAWPGLDWYEFE (SEQ ID NO: 15) as CDR3; or
f) the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3; or
g) the amino acid sequences (i) ETSGRHFDIDDMG (SEQ ID NO: 19) as CDR1, (ii) ITTESSTT (SEQ ID NO: 20) as CDR2 and (iii) NAEMHPRSLDYALGNRD (SEQ ID NO: 21) as CDR3; or
h) the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3; or
i) the amino acid sequences (i) AASGRTHDWYTMG (SEQ ID NO: 25) as CDR1, (ii) INWSSGMTY (SEQ ID NO: 26) as CDR2 and (iii) NVHPFTSPD (SEQ ID NO: 27) as CDR3; or
j) the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3, or
k) amino acid sequences that have at least 90% sequence homology with the amino acid sequences as defined in a), b), c), d), e), f), g), h), i), or j).,
wherein the nanobody binds to the Receptor binding domain (RBD) of SARS-CoV-2.

The novel nanobodies according to the invention bind with high affinities to the glycosylated SARS-CoV-2 spike protein receptor domain (RBD) and effectively block the interaction between RBD, S1-domain and trimeric spike protein with the viral docking site on human cells, the angiotensin converting enzyme 2 (ACE2). The inventors were able to show that the nanobodies of the invention have a neutralizing effect with IC₅₀ values in the low nanomolar range in a cell-based SARS-CoV-2 neutralization assay.

Accordingly, the novel nanobodies have a high potential for prophylactic as well as therapeutic options and provide a novel approach to screen for a neutralizing immune response in infected or vaccinated individuals thus helping to monitor the immune status or to guide vaccine design.

The nanobodies of the invention are neutralizing single domain antibodies preventing cellular entry by binding to the docking site of the SARS-CoV-2. The virus binds to the angiotensin converting enzyme II (ACE2) exposed on human lung epithelial via its receptor binding domain (RBD) located within the homotrimeric transmembrane spike glycoprotein (Spike).

As single domain antibodies they represent an excellent alternative to conventional antibodies (IgGs): Antibodies from camelids, such as llamas, include a unique subset of immunoglobulins consisting of heavy chain homodimers devoid of light chains. Their variable region (V_{H}H) is the smallest antigen-binding fragment found in the antibody world, and as a single polypeptide chain it is especially suitable for protein engineering. Single domain antibodies, or "nanobodies", are the recombinant minimal-sized, intact antigen-binding domains derived from the V_{H}H region of these heavy-chain antibodies. Unlike monoclonal antibodies, they can be readily produced in large amounts in simple bacterial expression systems. Moreover, nanobodies are usually extremely stable, can bind antigens with affinities in the nanomolar range, and are smaller in size (approximately 15 kDa) and thereby easier to manipulate genetically as compared with antibody fragments such as ScFvs.

Also, due to the nanobodies' small size and compact folding they show a high chemical stability, solubility and fast tissue penetration. Additionally, the nanobodies be easily converted into multivalent formats, such as bivalent, trivalent, tetravalent or other multi-specific constructs, e.g. addressing different epitopes on the same antigen, which the nanobodies presented herein can also be used for according to the invention. Also, nanobodies have comparable antigen specificities and affinities and, due to their high homology with human antibody (VH) fragments, show only very low immunogenicity.

However, to date only very few nanobodies were identified displaying a high affinity in the monovalent format. Most of them have to be converted into multivalent formats or applied as Fc fusion to efficiently block virus entry. This limits the developability and applicability of those binders since protein size is distinctly increased and furthermore Fc fusions bear the risk of unwanted antibody-dependent enhancement (ADE) in patients. Additionally, the observed high mutation rate of SARS-CoV-2 further stresses the need of selecting high affinity nanobodies addressing multiple epitopes within the RBD to ensure sufficient neutralization potency of virus harboring sequential and/ or structural changes in their docking site.

The nanobodies presented according to the invention display a high affinity in the monovalent format, and, thus, can efficiently block virus entry in the nM range.

According to a preferred embodiment, the nanobody of the invention, in its monovalent format, has a K_{D} of lower than 60 nM, 50 nM, 40nM, 30 nM, 20nM, 15 nM, 10 nM, 9 nM, 8nM, 7 nM, 6 nM, 5 nM, 4 nM, 3 nM, 2 nM, 1 nM.

According to a preferred embodiment, the nanobody of the invention, in its monovalent format, has a K_{D} of between 60 nM and 1 nM, 50 nM and 1 nM, 40nM and nM, 30 nM and 1 nM, 20nM and 1nM, 15 nM and 1 nM, 10 nM and 1nM, 9 nM and 1 nM, 8nM and 1nM, 7 nM and 1 nM, 6 nM and 1 nM, 5 nM and 1 nN, 4 nM and 1 nM, 3 nM and nM, 2 nM and 1 nM, or 1 nM and 0,5 nM.

The K_{D}, i.e. the dissociation constant, is an equilibrium constant that measures the propensity of a larger object, e.g. complex, to separate (dissociate) reversibly into its component molecules: In order for a drug to exert its pharmacological effects, the active ingredients it contains must (generally) bind to molecular target structures in the organism, the so-called drug targets. These include, e.g. receptors, enzymes, transporters and ion channels, and, in the present invention, the target represent angiotensin converting enzyme II (ACE2) expressed on human tissue. This binding is formally represented for receptors in this way: D(rug) + R(eceptor) ⇄ Drug-Receptor complex (DR).

As mentioned above, the dissociation constant is an equilibrium constant that indicates where the equilibrium of this process lies and indicates the ratio of bound and unbound active ingredient. It is defined as follows (C = concentration): K_{D} = C(D) - C(R) / C(DR). The unit of K_{D} is mol/L and can also be indicated with M. The smaller the value, the more active ingredient is bound to the receptor and the higher the binding affinity of the active ingredient. The strongest possible binding is usually also a prerequisite for effectiveness.

The dissociation constant of the nanobody can be determined or measured by several determination methods known to one skilled in the art, and its characterization is not bound to a specific determination method. The K_{D} of a binding pair, such as the nanobody and its binding target, can be assessed using surface-based (heterogeneous) methods including surface plasmon resonance (SPR) (see, e.g.: Schuck P., "Reliable determination of binding affinity and kinetics using surface plasmon resonance biosensors", Current Opinion in Biotechnology 8, 498-502 (1997)), biolayer interferometry (BLI) (see, e.g. Gauglitz, G., "Direct optical detection in bioanalysis: an update", Analytical and Bioanalytical Chemistry 398, 2363-2372, (2010)) and enzyme linked immunosorbent assays (ELISA) (Friguet et al., "Measurements of the true affinity constant in solution of antigen-antibody complexes by enzyme-linked immunosorbent assay", Journal of Immunological Methods 77, 305-319 (1985). In the present invention, biolayer interferometry was used.

In the context of the present invention, the terms of "antibody" and "immunoglobulin" have the same meaning and are used indifferently. In conventional antibodies, the two heavy chains are bound to each other through disulfide bridges and each heavy chain is bound to a light chain through a disulfide bridge. There are two types of light chain: lambda (λ) and kappa (κ) light chains. There exist five main classes of heavy chains (or isotypes) which determine the functional activity of an antibody: IgM, IgD, IgG, IgA and IgE. Each chain contains distinct sequence domains. The light chain comprises two domains: a variable domain (VL) and a constant domain (CL). The heavy chain comprises four domains: a variable domain (VH) and three constant domains (CH1, CH2 and CH3, collectively designated as CH). The variable regions of the heavy chains (VH) and light chains (VL) determine the recognition of a bond and the specificity to the antigen. The domains of the constant regions of the light (CL) and heavy (CH) chains give important biological properties such as the association of the chains of the antibodies, secretion, transplacental mobility, the bond to the complement and the bond to Fc receptors. The fragment Fv is the N-terminal portion of the Fab fragment of an immunoglobulin consisting of variable portions of a light chain and of a heavy chain. The specificity of the antibody lies in the structural complementarity between the combination site of the antibody and the antigenic determinant. The combination sites of the antibody are made of residues which mainly stem from hypervariable regions or regions for determining complementarity (CDRs). Occasionally, residues from non-hypervariable regions or "framework" regions (FR) may influence the global structure of the domain and therefore the combination site.

Within the context of the invention, the term "CDR" refers to the sequences of amino acids, which together define the bond affinity and the specificity of the natural Fv region of a native binding site of an immunoglobulin. The heavy and light chains of an immunoglobulin each have three CDRs, designated as H-CDR1, H-CDR2, H-CDR3 and L-CDR1, L-CDR2, L-CDR3 respectively.

Within the context of the invention, the terms of "framework region", 'framework" or "FR", refer to amino acid sequences inserted between the CDRs.

Within the context of the invention, the terms of "nanobody", "VHH", "VHH antibody fragment" and "single domain antibody" are used indifferently and designate the variable domain of the single heavy chain of antibodies of the type of those found in *Camelidae,* which are naturally without any light chains. In the absence of a light chain, the nanobodies each have three CDRs, designated as CDR1, CDR2 and CDR3 respectively. The nanobodies according to the invention may in particular be nanobodies of camels, dromedaries, llamas or alpacas. Preferably, the nanobodies according to invention are nanobodies of alpacas.

By "nanobody directed against SARS-CoV-2", is meant here a nanobody capable of selectively binding to the Receptor binding domain (RBD) of SARS-CoV-2. Preferentially, the nanobody is specific of RBD of SARS-CoV-2, i.e. it binds to RBD of SARS-CoV-2 excluding any other molecule.

The present inventors have more specifically identified 10 nanobodies directed against the RBD of SARS-CoV-2 and having unexpected features, in particular a very strong affinity for RBD of SARS-CoV-2.

A nanobody having high (i.e., 90% or greater) homology to the preferred amino acid sequences as set forth above can be obtained by mutagenesis according to any method known in the art or disclosed herein (e.g., site-directed or PCR-mediated mutagenesis of nucleic acid molecules encoding, e.g., any of the sequences set forth herein (i.e. SEQ ID NO:1 to SEQ ID NO: 41, with each and every number of the SEQ ID Nos., although here not specifically listed, being encompassed by this range), followed by testing of the encoded altered nanobody for retention of one or more desired features/properties as set forth above, e.g., using a functional assay as known in the art or described herein.

The percent homology between two amino acid sequences is equivalent to the percent identity between the two sequences. The percent identity between the two sequences is a function of the number of identical positions shared by the sequences (i.e., % homology=(number of identical positions)/(total number of positions)×100), taking into account the number of gaps, and the length of each gap, which need to be introduced for optimal alignment of the two sequences as known in the art.

A non-limiting example of a method by which the homology or % identity between two sequences can be determined is the algorithm of E. Meyers and W. Miller (Comput Appl Biosci, 4(1988), 11-17) which has been incorporated into the ALIGN program (version 2.0), using a PAM120 weight residue table, a gap length penalty of 12 and a gap penalty of 4. In addition, the percent identity between two amino acid sequences can also be determined using the Needleman and Wunsch (J Mol Biol 48(1970):444-453) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6.

Thus, the amino acid sequences of the invention, which have a homology of at least about 90% to the amino acid sequences as specifically disclosed and listed herein, represent "homologs" or "variants" of the nanobodies disclosed herein are also provided. Homologs and variants of the amino acid sequences disclosed herein, e.g., of a nanobody disclosed herein that specifically binds to the RBD of SARS-CoV-2, are typically characterized by having at least about 90%, 95%, 96%, 97%, 98% or 99% sequence identity as determined over the full length alignment with the specific amino acid sequence as set forth herein for/of the nanobody, e.g., as determined using the NCBI Blast 2.0 and as otherwise known in the art. One of skill in the art will appreciate that these sequence identity ranges are provided for guidance only; it is entirely possible that strongly significant homologs could be obtained that fall outside of the ranges provided.

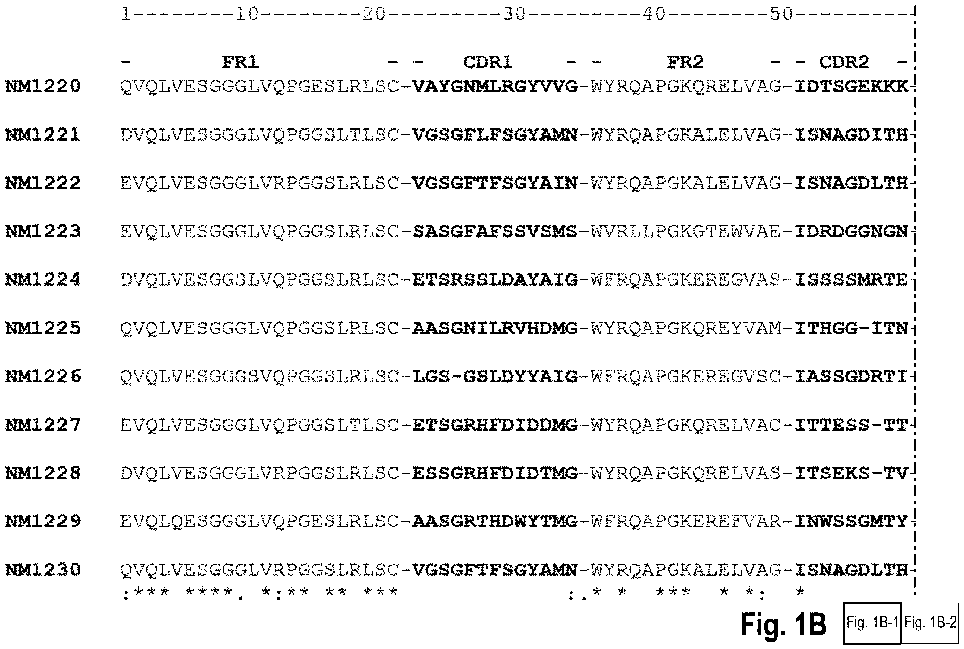

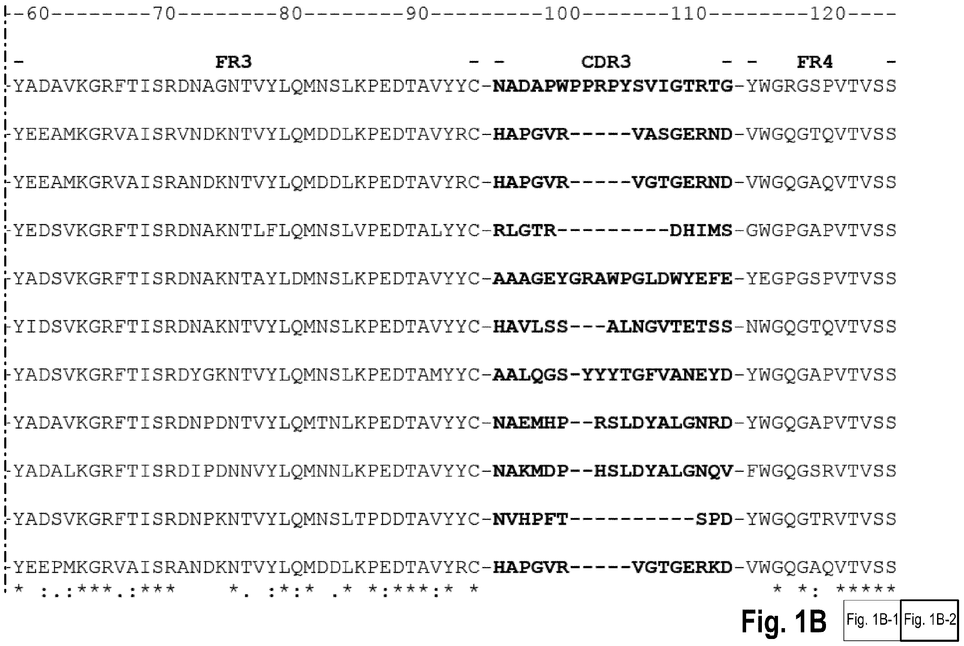

The amino acid sequences of the 10 antibodies are given in Fig. 1B. Accordingly, nanobody NM1220 has the amino acid sequence QVQLVESGGGLVQPGESLRLSCVAYGNMLRGYVVGWYRQAPGKQRELVAGIDTSGEK KKYADAVKGRFTISRDNAGNTVYLQMNSLKPEDTAVYYCNADAPWPPRPYSVIGTRTGY WGRGSPVTVSS (SEQ ID NO: 31), the nanobody NM1221 has the amino acid sequence DVQLVESGGGLVQPGGSLTLSCVGSGFLFSGYAMNWYRQAPGKALELVAGISNAGDIT HYEEAMKGRVAISRVNDKNTVYLQMDDLKPEDTAVYRCHAPGVRVASGERNDVWGQG TQVTVSS (SEQ ID NO: 32), the nanobody NM1222 has the amino acid sequence EVQLVESGGGLVRPGGSLRLSCVGSGFTFSGYAINWYRQAPGKALELVAGISNAGDLTH YEEAMKGRVAISRANDKNTVYLQMDDLKPEDTAVYRCHAPGVRVGTGERNDVWGQGA QVTVSS (SEQ ID NO: 33), the nanobody NM1223 has the amino acid sequence EVQLVESGGGLVQPGGSLRLSCSASGFAFSSVSMSWVRLLPGKGTEWVAEIDRDGGN GNYEDSVKGRFTISRDNAKNTLFLQMNSLVPEDTALYYCRLGTRDHIMSGWGPGAPVTV SS (SEQ ID NO. 34), the nanobody NM1224 has the amino acid sequence DVQLVESGGSLVQPGGSLRLSCETSRSSLDAYAIGWFRQAPGKEREGVASISSSSMRTE YADSVKGRFTISRDNAKNTAYLDMNSLKPEDTAVYYCAAAGEYGRAWPGLDWYEFEYE GPGSPVTVSS (SEQ ID NO. 35), the nanobody NM1226 has the amino acid sequence QVQLVESGGGLVQPGGSLRLSCAASGNILRVHDMGWYRQAPGKQREYVAMITHGGITN YIDSVKGRFTISRDNAKNTVYLQMNSLKPEDTAVYYCHAVLSSALNGVTETSSNWGQGT QVTVSS (SEQ ID NO. 36), the nanobody NM1227 has the amino acid sequence EVQLVESGGGLVQPGGSLTLSCETSGRHFDIDDMGWYRQAPGKQRELVACITTESSTTY ADAVKGRFTISRDNPDNTVYLQMTNLKPEDTAVYYCNAEMHPRSLDYALGNRDYWGQG APVTVSS (SEQ ID NO. 37), the nanobody NM1228 has the amino acid sequence DVQLVESGGGLVRPGGSLRLSCESSGRHFDIDTMGWYRQAPGKQRELVASITSEKSTV-YADALKGRFTISRDIPDNNVYLQMNNLKPEDTAVYYCNAKMDPHSLDYALGNQVFWGQ GSRVTVSS (SEQ ID NO. 38), the nanobody NM1229 has the amino acid sequence EVQLQESGGGLVQPGESLRLSCAASGRTHDWYTMGWFRQAPGKEREFVARINWSSG MTYYADSVKGRFTISRDNPKNTVYLQMNSLTPDDTAVYYCNVHPFTSPDYWGQGTRVT VSS (SEQ ID NO. 39), and the nanobody NM1230 has the amino acid sequence QVQLVESGGGLVRPGGSLRLSCVGSGFTFSGYAMNWYRQAPGKALELVAGISNAGDLT HYEEPMKGRVAISRANDKNTVYLQMDDLKPEDTAVYRCHAPGVRVGTGERKDVWGQG AQVTVSS (SEQ ID NO. 40).

The CDRs of these ten nanobodies are the following:
NM1220: CDR1: VAYGNMLRGYVVG (SEQ ID NO: 1) CDR2: IDTSGEKKK (SEQ ID NO: 2) CDR3: NADAPWPPRPYSVIGTRTG (SEQ ID NO: 3); NM1221: CDR1: VGSGFLFSGYAMN (SEQ ID NO: 4) CDR2: ISNAGDITH (SEQ ID NO: 5) CDR3: HAPGVRVASGERND (SEQ ID NO.6); NM1222: CDR1: VGSGFTFSGYAIN (SEQ ID NO: 7) CDR2: ISNAGDLTH (SEQ ID NO: 8) CDR3: HAPGVRVGTGERND (SEQ ID NO. 9; NM1223: CDR1: SASGFAFSSVSMS (SEQ ID NO: 10) CDR2: IDRDGGNGN (SEQ ID NO: 11) CDR3: RLGTRDHIMS (SEQ ID NO. 12); NM1224: CDR1: ETSRSSLDAYAIG (SEQ ID NO. 13) CDR2: ISSSSMRTE (SEQ ID NO. 14) CDR3: AAAGEYGRAWPGLDWYEFE (SEQ ID NO: 15); NM1226: CDR1: LGSGSLDYYAIG (SEQ ID NO: 16) CDR2: IASSGDRTI (SEQ ID NO. 17) CDR3: AALQGSYYYTGFVANEYD (SEQ ID NO. 18); NM1227: CDR1: ETSGRHFDIDDMG (SEQ ID NO. 19) CDR2: ITTESSTT (SEQ ID NO. 20) CDR3: NAEMHPRSLDYALGNRD (SEQ ID NO. 21); NM1228: CDR1: ESSGRHFDIDTMG (SEQ ID NO. 22) CDR2: ITSEKSTV (SEQ ID NO. 23) CDR3: NAKMDPHSLDYALGNQV (SEQ ID NO. 24); NM1229: CDR1: AASGRTHDWYTMG (SEQ ID NO. 25) CDR2: INWSSGMTY (SEQ ID NO. 26) CDR3: NVHPFTSPD (SEQ ID NO. 27); NM1230: CDR1: VGSGFTFSGYAMN (SEQ ID NO. 28) CDR2: ISNAGDLTH (SEQ ID NO. 29) CDR3: HAPGVRVGTGERKD (SEQ ID NO. 30).

As this is well known to one skilled in the art, the combination of the CDR1, CDR2 and CDR3 is sufficient for defining a site for binding to the antigen. Therefore, an object of the present invention relates to a nanobody directed against SARS-CoV-2 comprising the a) the amino acid sequences (i) VAYGNMLRGYVVG (SEQ ID NO: 1) as CDR1, (ii) IDTSGEKKK (SEQ ID NO: 2) as CDR2 and (iii) NADAPWPPRPYSVIGTRTG (SEQ ID NO: 3) as CDR3; or b) the amino acid sequences (i) VGSGFLFSGYAMN (SEQ ID NO: 4) as CDR1, (ii) ISNAGDITH (SEQ ID NO: 5) as CDR2 and (iii) HAPGVRVASGERND (SEQ ID NO: 6) as CDR3; or c) the amino acid sequences (i) VGSGFTFSGYAIN (SEQ ID NO: 7) as CDR1, (ii) ISNAGDLTH (SEQ ID NO: 8) as CDR2 and (iii) HAPGVRVGTGERND (SEQ ID NO: 9) as CDR3 ; or d) the amino acid sequences (i) SASGFAFSSVSMS (SEQ ID NO: 10) as CDR1, (ii) IDRDGGNGN (SEQ ID NO: 11) as CDR2 and (iii) RLGTRDHIMS (SEQ ID NO: 12) as CDR3; or e) the amino acid sequences (i) ETSRSSLDAYAIG (SEQ ID NO: 13) as CDR1, (ii) ISSSSMRTE (SEQ ID NO: 14) as CDR2 and (iii) AAAGEYGRAWPGLDWYEFE (SEQ ID NO: 15) as CDR3; or f) the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3; or g) the amino acid sequences (i) ETSGRHFDIDDMG (SEQ ID NO: 19) as CDR1, (ii) ITTESSTT (SEQ ID NO: 20) as CDR2 and (iii) NAEMHPRSLDYALGNRD (SEQ ID NO: 21) as CDR3; or h) the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3; or i) the amino acid sequences (i) AASGRTHDWYTMG (SEQ ID NO: 25) as CDR1, (ii) INWSSGMTY (SEQ ID NO: 26) as CDR2 and (iii) NVHPFTSPD (SEQ ID NO: 27) as CDR3; or j) the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3, or a functionally conservative variant of the nanobody defined in a), b), c), d), e), f), g), h), i), j) comprising a conservative substitution of one or two amino acids in one, two or three of the sequences respectively SEQ ID NO: 1, SEQ ID NO: 2 and SEQ ID NO: 3, or SEQ ID NO: 4, SEQ ID NO: 5 and SEQ ID NO: 6, or SEQ ID NO: 7, SEQ ID NO: 8 and SEQ ID NO: 9, or SEQ ID NO: 10, SEQ ID NO: 11 and SEQ ID NO: 12.

The inventors also sequenced the "framework" regions (FR) of these nanobodies. The corresponding sequences are the following:
NM1220: Region FR1: QVQLVESGGGLVQPGESLRLSC (SEQ ID NO: 41) Region FR2: WYRQAPGKQRELVAG (SEQ ID NO: 42) Region FR3: YADAVKGRFTISRDNAGNTVYLQMNSLKPEDTAVYYC (SEQ ID NO: 43) Region FR4: YWGRGSPVTVSS (SEQ ID NO. 44); NM1221 Region FR1: DVQLVESGGGLVQPGGSLTLSC (SEQ ID NO: 45) Region FR2: WYRQAPGKALELVAG (SEQ ID NO: 46) Region FR3: YEEAMKGRVAISRVNDKNTVYLQMDDLKPEDTAVYRC (SEQ ID NO: 47) Region FR4: VWGQGTQVTVSS (SEQ ID NO. 48); NM1222: Region FR1: EVQLVESGGGLVRPGGSLRLSC (SEQ ID NO: 49) Region FR2: WYRQAPGKALELVAG (SEQ ID NO: 50) Region FR3: YEEAMKGRVAISRANDKNTVYLQMDDLKPEDTAVYRC (SEQ ID NO: 51) Region FR4: VWGQGAQVTVSS (SEQ ID NO. 52); NM1223 Region FR1: EVQLVESGGGLVQPGGSLRLSC (SEQ ID NO: 53) Region FR2: WVRLLPGKGTEWVAE (SEQ ID NO: 54) Region FR3: YEDSVKGRFTISRDNAKNTLFLQMNSLVPEDTALYYC (SEQ ID NO: 55) Region FR4: GWGPGAPVTVSS (SEQ ID NO. 56); NM1224: Region FR1: DVQLVESGGSLVQPGGSLRLSC (SEQ ID NO. 57) Region FR2: WFRQAPGKEREGVAS (SEQ ID NO. 58) Region FR3: YADSVKGRFTISRDNAKNTAYLDMNSLKPEDTAVYYC (SEQ ID NO. 59) Region FR4: YEGPGSPVTVSS (SEQ ID NO. 60); NM1226: Region FR1: QVQLVESGGGSVQPGGSLRLSC (SEQ ID NO. 61) Region FR2: WFRQAPGKEREGVSC (SEQ ID NO: 62) Region FR3: YADSVKGRFTISRDYGKNTVYLQMNSLKPEDTAMYYC (SEQ ID NO: 63) Region FR4: YWGQGAPVTVSS (SEQ ID NO. 64); NM1227: Region FR1: EVQLVESGGGLVQPGGSLTLSC (SEQ ID NO. 65) Region FR2: WYRQAPGKQRELVAC (SEQ ID NO. 66) Region FR3: YADAVKGRFTISRDNPDNTVYLQMTNLKPEDTAVYYC (SEQ ID NO. 67) Region FR4: YWGQGAPVTVSS (SEQ ID NO. 68); NM1228: Region FR1: DVQLVESGGGLVRPGGSLRLSC (SEQ ID NO. 69) Region FR2: WYRQAPGKQRELVAS (SEQ ID NO. 70) Region FR3: YADALKGRFTISRDIPDNNVYLQMNNLKPEDTAVYYC (SEQ ID NO. 71) Region FR4: FWGQGSRVTVSS (SEQ ID NO. 72); NM1229: Region FR1: EVQLQESGGGLVQPGESLRLSC (SEQ ID NO: 73) Region FR2: WFRQAPGKEREFVAR (SEQ ID NO. 74) Region FR3: YADSVKGRFTISRDNPKNTVYLQMNSLTPDDTAVYYC (SEQ ID NO. 75) Region FR4: YWGQGTRVTVSS (SEQ ID NO. 76); NM1230: Region FR1: QVQLVESGGGLVRPGGSLRLSC (SEQ ID NO. 77) Region FR2: WYRQAPGKALELVAG (SEQ ID NO. 78) Region FR3: YEEPMKGRVAISRANDKNTVYLQMDDLKPEDTAVYRC (SEQ ID NO. 79) Region FR4: VWGQGAQVTVSS (SEQ ID NO. 90).

In a preferred embodiment, the invention relates to a nanobody comprising or consisting in the chaining of sequences FR1-CDR1-FR2-CDR2-FR3-CDR3-FR4 as defined above of one of the nanobodies identified by the inventors.

Preferably, the nanobody according to the invention is therefore a nanobody comprising or consisting of a sequence of amino acids selected from the group consisting of the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39 and SEQ ID NO: 40, or a functionally conservative variant of the latter, having a K_{D} of lower than 60 nM, and comprising a conservative substitution of one or two amino acids in one, two or three of the CDRs comprised in the sequence of amino acids respectively SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39 and SEQ ID NO: 40. The functionally conservative variant as defined above may further comprise of one or several substitutions, in particular one or several conservative substitutions in the regions of the amino acid sequences respectively which are not CDRs, such as the "framework" regions, in particular the "framework" regions defined above.

In the context of the invention, the expression "functionally conservative variant" refers to variants in which a given amino acid in a nanobody according to the invention is substituted without altering the global conformation and the function of the nanobody (having a K_{D} of lower than 60 nM) including a replacement of an amino acid with another having similar properties (for example polarity, hydrogen bond potential, acidity, basicity, hydrophobicity, presence of an aromatic group, etc). The amino acids having similar properties are well known to one skilled in the art, for example arginine, histidine and lysine from hydrophilic-basic amino acids and may be interchangeable. Similarly, isoleucine, a hydrophobic amino acid, may be replaced with leucine, methionine or valine. Such changes should have no or little effect on the apparent molecular weight or on the isoelectric point of the nanobody. A natural amino acid may be replaced with a non-natural amino acid, such as an amino acid in a D configuration, a beta or gamma amino acid.

According to one aspect of the invention, the nanobody of the invention and as described herein can be used as a drug or medicament, either alone or in combination with at least one other nanobody of the invention, or in combination with another active ingredient or accessory moiety.

According to a preferred embodiment, the nanobody of the invention is used in the therapy or prophylaxis of a SARS-CoV-2-infection, wherein the nanobody is used alone or in combination with at least one another substance or accessory moiety, which is selected from a therapeutically active substance and/or at least one another nanobody of the invention. In a preferred embodiment, a patient, in particular a human, that has or is suspected of having a SARS-CoV-2-infection, is treated with the nanobody of the invention, a polypeptide comprising the nanobody of the invention, and/or a pharmaceutical composition comprising the nanobody of the invention, and optionally other accessory moieties, by administering the nanobody, the peptide and/or the pharmaceutical composition of the invention to said patient.

In embodiments, where the antibody is used in combination with a therapeutically active substance or with another nanobody of the invention, the nanobody and the substance are present in the combination as two separate entities which are not linked via a linker or spacer or any other connecting means. In this embodiment, the at least one another substance is used in addition to the nanobody, e.g. to support, increase or otherwise modify its function or effect in the therapy. E.g. the nanobody and the at least one another substance can be present - as separate entities - in one pharmaceutical composition for administration, or they can be used/administered subsequently. E.g., an antibody having the amino acid sequence of SEQ ID NO. SEQ ID NO: 31 is used in combination with e.g. an antibody having SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39 and/or SEQ ID NO: 40, whereby any combination of these sequences is possible and disclosed, also a combination of more than two nanobodies as disclosed herein.

According to another embodiment, the nanobody and at least one another substance, and/or nanobody, when used in combination, are linked to each other, preferably directly, or indirectly, preferably via a linker. Such embodiments represent a conjugate-construct, comprising a nanobody as disclosed herein linked to an accessory moiety.

As used in the context of the conjugate construct, the term "linked" may refer to the covalent linkage of the accessory moiety to the nanobody or may refer to noncovalent linkage of the accessory moiety to the nanobody. The accessory moiety may be a biologically active moiety (BAM), which exhibits one or more activity on a biological system rendering the BAM or conjugate-construct suitable for the treatment, prevention or amelioration of a SARS-CoV-2-infection, or a symptom thereof. Alternatively, or additionally, the accessory moiety may exhibit no detectable biological activity, but may function as a signal or reporter moiety suitable to allow the detection of the accessory moiety or the conjugate-construct *in vitro* or *in vivo,* e.g., to aid in the screening or diagnosis of antibodies or a SARS-CoV-2-infection. Further, the accessory moiety may also itself be a conjugating molecule, enabling additional covalent or non-covalent binding to further target molecules. Such conjugating accessory molecules may enable isolation of the conjugate-construct and/or screening and diagnostic methods, e.g., by binding to further signal or reporter molecules. Non-limiting examples of such conjugating accessory molecules include, e.g., biotin and hexahistidine tags as known in the art.

Where the accessory moiety is also a protein, peptide or polypeptide, it may be conjugated to the nanobody to form a conjugate-construct via a peptide-bond. The accessory moiety may be chemically conjugated to the nanobody directly or may be linked to the nanobody through a linker group. As used throughout the disclosure, direct conjugation indicates the conjugation of the accessory moiety to any amino acid residue within nanobody using any chemical coupling known in the art or described herein suitable for the conjugation of the accessory moiety to an amino acid residue (e.g., an amino acid side chain) of the nanobody. Accordingly, direct coupling may result in one or more chemical groups spaced between the accessory moiety and the amino acid (e.g., amino acid side chain) of the nanobody, which groups form as a result of the coupling reaction as is known in the art.

Alternatively, as described herein, the accessory moiety may be conjugated to any amino acid residue within the nanobody indirectly, that is, via a linker group. Therefore, as used throughout this disclosure, indirect conjugation means that the accessory moiety is conjugated to the linker group, which linker group is conjugated to an amino acid residue within the nanobody. The conjugation between the accessory moiety and the linker group and between the linker group and an amino acid residue of the nanobody may be any conjugation method and/or compound suitable for effecting such conjugation as described herein or as is otherwise known in the art. The conjugation between the accessory molecule and the nanobody, whether direct or indirect, may be via a cleavable or non-cleavable linker.

The direct or indirect conjugation of the accessory moiety may be directed to any amino acid residue within the nanobody as described herein. Thus, the accessory moiety may be directly or indirectly conjugated to an amino acid residue that is at the N or C terminus of the nanobody. Alternatively or additionally, the accessory moiety may be directly or indirectly conjugated to an internal amino acid residue of the nanobody. As is known in the art, conjugation methods (whether direct or indirect) may require the chemical modification of one or both sites of conjugation (e.g., modification of an amino acid residue within or at the terminus of the linker group, accessory molecule, and/or the nanobody disclosed herein). Accordingly, the present invention also encompasses chemical modification of the components of the conjugate-constructs disclosed herein (e.g., the linker group, accessory molecule, and/or the nanobody) described herein suitable to allow conjugation of said compounds and components. Where a linker group is present, such linker group may be any linker, e.g., a peptide linker, known in the art or disclosed herein suitable for linking the nanobody to the accessory moiety. Non-limiting examples of linker groups include peptide linkers, e.g., comprising one or more residues of glutamic acid, glycine, serine, cysteine and combinations thereof.

The invention also encompasses conjugate-constructs wherein the accessory moiety is directly linked to the nanobody. Where the conjugate-construct is lacking a linking group, the accessory moiety may be conjugated, e.g., chemically conjugated, directly to a residue within or at the terminus of the nanobody's amino acid sequence. Non-limiting examples of such chemical conjugation include covalent attachment to the peptide molecule at the N-terminus and/or to the N-terminal amino acid residue via an amide bond or at the C-terminus and/or C-terminal amino acid residue via an ester bond.

Accordingly, if one nanobody of the invention is linked to another nanobody of the invention, the nanobody has a bivalent format, and if, e.g., three nanobodies of the invention are linked together, the nanobody has a trivalent format. Such formats can be desirable to enhance or otherwise modify the effectiveness of the nanobody of the invention.

According to a preferred embodiment, a nanobody, e.g. the nanobody for use as disclosed herein, which comprises the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3 is used in combination with at least one another nanobody as disclosed herein.

According to another preferred embodiment, a nanobody comprising the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3, or a nanobody comprising the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3 is used in the combination.

Accordingly, in a preferred embodiment, a nanobody, e.g. the nanobody for use as disclosed herein, which comprises the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3 is used in combination with a nanobody comprising the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3, or a nanobody comprising the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3.

In yet another preferred embodiment of the nanobody, the methods and uses of the invention and disclosed herein, a nanobody having the amino acid sequence SEQ ID NO: 40 (NM1230) is combined with a nanobody having one of the following amino acid sequences: SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, and SEQ ID NO: 39.

Accordingly, preferred embodiments are a combination of a nanobody having (or comprising or consisting of) the amino acid sequence SEQ ID NO: 40 (NM1230) with a nanobody having the following amino acid sequence: SEQ ID NO: 31 (NM1220), a combination of a nanobody having the amino acid sequence SEQ ID NO: 40 (NM1230) with a nanobody having the following amino acid sequence: SEQ ID NO: 32 (NM1221), a combination of a nanobody having the amino acid sequence SEQ ID NO: 40 (NM1230) with a nanobody having the following amino acid sequence: SEQ ID NO: 33 (NM1222), a combination of a nanobody having the amino acid sequence SEQ ID NO: 40 (NM1230) with a nanobody having the following amino acid sequence: SEQ ID NO: 34 (NM1223), a combination of a nanobody having the amino acid sequence SEQ ID NO: 40 (NM1230) with a nanobody having the following amino acid sequence: SEQ ID NO: 35 (NM1224), a combination of a nanobody having the amino acid sequence SEQ ID NO: 40 (NM1230) with a nanobody having the following amino acid sequence: SEQ ID NO: 36 (NM1226), a combination of a nanobody having the amino acid sequence SEQ ID NO: 40 (NM1230) with a nanobody having the following amino acid sequence: SEQ ID NO: 37 (NM1227), a combination of a nanobody having the amino acid sequence SEQ ID NO: 40 (NM1230) with a nanobody having the following amino acid sequence: SEQ ID NO: 38 (NM1228), a combination of a nanobody having the amino acid sequence SEQ ID NO: 40 (NM1230) with a nanobody having the following amino acid sequence: SEQ ID NO: 39 (NM1229).

Additionally preferred combinations of nanobodies of the invention are the following: a combination of a nanobody comprising or consisting of an amino acid sequence having SEQ ID NO. 38 (NM1228) with a nanobody comprising or consisting of an amino acid sequence having SEQ ID NO. 32 (NM1221), a combination of a nanobody comprising or consisting of an amino acid sequence having SEQ ID NO. 38 (NM1228) with a nanobody comprising or consisting of an amino acid sequence having SEQ ID NO. 33 (NM1222), a combination of a nanobody comprising or consisting of an amino acid sequence having SEQ ID NO. 38 (NM1228) with a nanobody comprising or consisting of an amino acid sequence having SEQ ID NO. 34 (NM1223), a combination of a nanobody comprising or consisting of an amino acid sequence having SEQ ID NO. 38 (NM1228) with a nanobody comprising or consisting of an amino acid sequence having SEQ ID NO. 35 (NM1224), a combination of a nanobody comprising or consisting of an amino acid sequence having SEQ ID NO. 38 (NM1228) with a nanobody comprising or consisting of an amino acid sequence having SEQ ID NO. 31 (NM1220), a combination of a nanobody comprising or consisting of an amino acid sequence having SEQ ID NO. 40 (NM1230) with a nanobody comprising or consisting of an amino acid sequence having SEQ ID NO. 34 (NM1223), a combination of a nanobody comprising or consisting of an amino acid sequence having SEQ ID NO. 40 (NM1230) with a nanobody comprising or consisting of an amino acid sequence having SEQ ID NO. 35 (NM1224), a combination of a nanobody comprising or consisting of an amino acid sequence having SEQ ID NO. 40 (NM1230) with a nanobody comprising or consisting of an amino acid sequence having SEQ ID NO. 31 (NM1220), a combination of a nanobody comprising or consisting of an amino acid sequence having SEQ ID NO. 34 (NM1223) with a nanobody comprising or consisting of an amino acid sequence having SEQ ID NO. 35 (NM1224), a combination of a nanobody comprising or consisting of an amino acid sequence having SEQ ID NO. 34 (NM1223) with a nanobody comprising or consisting of an amino acid sequence having SEQ ID NO. 31 (NM1220), and a combination of a nanobody comprising or consisting of an amino acid sequence having SEQ ID NO. 35 (NM1224) with a nanobody comprising or consisting of an amino acid sequence having SEQ ID N. 31 (NM1220).

Also combinations of three nanobodies are suitable, in particular nanobody combinations of the nanobody having the amino acid sequence SEQ ID NO: 40 (NM1230) with at least two other nanobodies as disclosed herein.

It is to be understood that all combination, in particular the specifically listed combinations, can be used in any therapeutic, diagnostic, or prognostic method or use, in particular in any therapeutic, diagnostic, or prognostic method or use according to the invention, in particular in the ones disclosed therein.

According to a preferred embodiment, the nanobody of the invention is used alone or in combination with at least one other nanobody as disclosed herein for diagnostic or prognostic methods. The same combination principles for the nanobody of the invention as explained above for the therapeutic uses apply for the diagnostic or prognostic uses.

According to a preferred embodiment, the nanobody as disclosed herein/ of the invention is used in *in vitro* methods for screening of the emergence and/or presence of neutralizing SARS-CoV-2 antibodies in a biological sample.

With the nanobodies presented herein, a biological sample can be screened for antibodies that are neutralizing and prevent reinfection with SARS-CoV-2. While the testing systems existing in the prior art detect the overall antibody response against distinct antigens of SARS-CoV-2, they do not provide the answer to the most relevant question whether the tested individuals carry neutralizing antibodies.

E.g., the nanobodies of the invention can be employed with a ligand binding assay, e.g. with a competitive binding assay, where the nanobodies of the invention, in particular the combination of the nanobodies as mentioned above, are combined with immunoassays, preferably automatable multiplex immunoassays. Competition binding is used to determine the presence and selectivity of a particular ligand (herein: antibodies specific for the RBD). Competition curves are obtained by plotting specific binding, which is the percentage of the total binding, against the log concentration of the competing ligand

According to another embodiment, the nanobody of the invention is used alone or in combination with at least one other nanobody of the invention, for use in determining the presence or absence of SARS-CoV-2 in a biological sample.

In the diagnostic methods described herein, the biological sample to be assayed, e.g. a sample containing or suspected of containing SARS-Co-V-2 material, is contacted with a nanobody of the invention, or a combination of the nanobody with at least one another nanobody of the invention, or a combination with a nanobody with an accessory moiety (conjugate-construct), as disclosed herein, and binding of the nanobody or conjugate-construct to SARS-CoV-2 in the sample is detected. An increase in binding of the nanobody or conjugate-construct in the sample as compared to binding of the nanobody or conjugate-construct to a control sample confirms the presence of SARS-CoV-2 in the subject. In some examples, the control sample is a sample that has been determined to be SARS-CoV-2-free.

The nanobodies of the invention can be associated with or linked to a detectable marker, in particular when used in diagnostic or prognostic methods/uses.

By "nanobody associated with a detectable marker (or label)" or is meant here that the detectable marker/label is indirectly or directly bound to the nanobody, or is incorporated into the nanobody.

As used herein the terms "label", "marker" and variants thereof when used in the context of another protein or molecule refer to a detectable compound, composition or molecule that is conjugated directly or indirectly to a second molecule (in particular, a nanobody or conjugate-construct disclosed herein) to facilitate detection of the second molecule. Non-limiting examples of labels as known in the art include fluorescent tags, enzymatic linkages, and radioactive isotopes. In one example, a "labeled nanobody" refers to the direct or indirect conjugation of the detectable compound, composition or molecule to the nanobody. Additionally, or alternatively, the detectable compound, composition or molecule can be incorporated into the nanobody structure by means other than direct or indirect conjugation. An exemplary method of such incorporation is the replacement of an amino acid residue of the nanobody with a modified amino acid residue such that it becomes detectable (e.g., by radiolabeling). The label may be directly detectable or may be detectable only after contact with further compounds compositions or molecules. In one non-limiting example, the label may be the incorporation of a radiolabeled amino acid, which is directly detectable according to methods known in the art. In additional or alternative non-limiting examples, the label may be the attachment of biotinyl moieties to the nanobody, which are detectable following contact with marked avidin (for example, streptavidin containing a fluorescent marker or enzymatic activity that can be detected by optical or colorimetric methods as known in the art). Various methods of labeling proteins are known in the art and may be used. Examples of labels for polypeptides include, but are not limited to, the following: radioisotopes or radionucleotides (such as ³⁵S, ¹¹C, ¹³N, ¹⁵O, ¹⁸F, ¹⁹F, ⁹⁹TC, ¹³¹I, ³H, ¹⁴C, ¹⁵N, ⁹⁰Y, ⁹⁹Tc, ¹¹¹In and ¹²⁵I), fluorescent labels (such as fluorescein isothiocyanate (FITC), rhodamine, lanthanide phosphors), enzymatic labels (such as horseradish peroxidase, beta-galactosidase, luciferase, alkaline phosphatase), chemiluminescent markers, biotinyl groups, predetermined polypeptide epitopes recognized by a secondary reporter (such as a leucine zipper pair sequences, binding sites for secondary antibodies, metal binding domains, epitope tags), or magnetic agents (such as gadolinium chelates). In some embodiments, labels are attached by spacer arms of various lengths to reduce potential steric hindrance for the binding of the nanobody or the conjugate-construct disclosed herein.

A "biological sample" as used herein is meant to be a portion of a larger biological element. Preferably, the sample is a substance of biological origin. Examples of biological samples include, but are not limited thereto, portions of organs or tissues such as the kidney, the liver, heart, the lung, etc., the arteries, the veins, etc., the blood, e.g. whole blood, and its compounds such as the plasma, serum, the platelets, the subpopulations of blood cells etc., as well as saliva, sputum, or any sample retrieved from the mouth and pharynx region, also e.g. by using a mouth wash or rinse containing saliva or sputum samples. The biological sample, preferably, is from a mammal, preferably a human; the biological sample can be from an individual with known or unknown infection-status or from a vaccinated individual or non-vaccinated individual.

The present invention also relates to a pharmaceutical composition comprising at least one nanobody as defined herein in association with a pharmaceutically acceptable carrier.

The term "pharmaceutically" or "pharmaceutically acceptable" refers to molecular entities and compositions which do not produce secondary, allergic or other unfortunate reactions when they are administered to a mammal, in particular a human.

Within the context of the invention, the expression "pharmaceutically acceptable carrier" includes any solvent, dispersion medium, coating, antibacterial or antifungal agent, isotonic agent or absorption retardant, and the like. The use of such media and agents for the pharmaceutically active substances is well known to one skilled in the art. Except for the case when a conventional medium or agent is incompatible with the active ingredient, its use is contemplated in pharmaceutical compositions. Additional active ingredients may also be incorporated into the composition.

Any administration method, known to one skilled in the art, may be used for administrating the nanobody or the pharmaceutical composition according to the invention to the patient. In particular, the nanobody/the pharmaceutical composition may be for example administered orally, by inhalation or via a parenteral route (in particular by intravenous injection). When the parenteral route is selected, the nanobody/pharmaceutical composition may be in the form of injectable solutions and suspensions, packaged in ampoules or flasks. The parenteral administration forms are conventionally obtained by mixing the nanobody according to the invention with buffers, stabilizers, preservatives, solubilizing agents, isotonic agents and suspending agents. According to known techniques, these mixtures may be sterilized or packaged as intravenous injections. One skilled in the art may for example use a buffer based on phosphate salts as buffers. Examples of suspension agents include methylcellulose, acacia, and sodium carboxymethylcellulose. Examples of stabilizers include sodium sulfite and sodium metasulfite, and examples of preservatives include sodium p-hydroxybenzoate, sorbic acid, cresol and chlorocresol.

The administered amount of nanobodies/the pharmaceutical composition naturally depends on the administration route/method, on the size and/or on the weight of the patient, on the detection technique used, and on the nature of the accessory moiety which may be associated therewith.

The patient preferably is a human.

According to a preferred embodiment, the pharmaceutical composition of the invention comprises a nanobody comprising the amino acid sequences (i) VGSGFTFSGYAMN (SEQ ID NO: 31) as CDR1, (ii) ISNAGDLTH (SEQ ID NO: 32) as CDR2 and (iii) HAPGVRVGTGERKD (SEQ ID NO: 33) as CDR3 in combination with at least one other nanobody as disclosed herein.

It is particularly preferred, if the pharmaceutical composition of the invention, e.g. the one mentioned above, comprises a nanobody comprising the amino acid sequences (i) LGSGSLDYYAIG (SEQ ID NO: 19) as CDR1, (ii) IASSGDRTI (SEQ ID NO: 20) as CDR2 and (iii) AALQGSYYYTGFVANEYD (SEQ ID NO: 21) as CDR3, or a nanobody comprising the amino acid sequences (i) ESSGRHFDIDTMG (SEQ ID NO: 25) as CDR1, (ii) ITSEKSTV (SEQ ID NO: 26) as CDR2 and (iii) NAKMDPHSLDYALGNQV (SEQ ID NO: 27) as CDR3 in the combination.

In a preferred embodiment, the pharmaceutical composition a nanobody comprising or consisting of the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3 is used in combination with a nanobody comprising the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3, or a nanobody comprising the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3.

According to a preferred embodiment, a pharmaceutical composition is provided, which comprises a nanobody comprising or consisting of the amino acid sequence of SEQ ID NO. SEQ ID NO: 31, which preferably is used in combination with e.g. an antibody having SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39 and/or SEQ ID NO: 40, in particular the combination of SEQ ID NO: 40 with either SEQ ID NO: 36 or SEQ ID NO. 38.

As mentioned above for the use of the nanobody of the invention, also a method for screening of the emergence and/or presence of SARS-CoV-2-neutralizing antibodies in a biological sample is disclosed, wherein a nanobody as disclosed herein alone or in combination with at least one other nanobody or accessory moiety as disclosed herein is used in the method, as well as a method for determining the presence or absence of SARS-CoV-2 in a biological sample, wherein a nanobody as disclosed herein, alone or in combination with at least one other nanobody or accessory moiety as claimed herein, is used in the method. In the methods of the invention, a biological sample is contacted with the nanobody/combinations of the nanobody and binding of the nanobody to SARS-CoV-2 in the sample is determined.

In the methods of the invention, it is particularly preferred, if a nanobody comprising the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3 is used in combination with at least one another nanobody as claimed in claims 1 to 3, preferably in combination with a nanobody comprising the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3, or a nanobody comprising the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3. It is also preferred if a nanobody having the SEQ ID NO. 30 is combined with a nanobody having SEQ ID NO. 36 or SEQ ID NO. 38.

Any method or assay known in the field can be employed in the diagnostic and prognostic methods of the invention, e.g. ligand binding assays, immunoassays, competition binding assays, etc., for determining the presence of SARS-CoV-2/SARS-CoV-2-antigen(s) or neutralizing antibodies.

E.g., the method of the invention for screening of neutralizing SARS-CoV-2-antibodies in a biological sample can represent a competition comprising the following steps: (i) bringing into contact, *in vitro,* polypeptides representing SARS-CoV-2-antigen/s, e.g. the receptor binding domain (RBD) of the homotrimeric Spike protein of the SARS-CoV-2, the S1 domain of the homotrimeric Spike protein of the SARS-CoV-2 and/or the homotrimeric Spike protein of the SARS-CoV-2 with (a) a biological sample containing or suspected to contain neutralizing antibodies directed against SARS-CoV-2, and with (b) a nanobody as disclosed herein, alone or in combination with at least one other nanobody as disclosed herein, preferably in different concentrations/dilutions, to generate a reaction mixture, and (ii) detecting neutralizing SARS-CoV-2-antibodies, that are possibly present in the biological sample, which are bound to the SARS-CoV-2-antigens in the reaction mixture.

Depending on the presence/concentration of the neutralizing SARS-CoV-2-antibodies in the biological sample, the nanobodies of the invention which bind to immobilized RBD of SARS-CoV-2 get competitively displaced from the binding sites through the possibly present SARS-CoV-2-antibodies. As a consequence, it follows that the higher the concentration of neutralizing SARS-CoV-2-antibodies is in the biological sample, the more nanobodies are replaced at the RBD binding site by the neutralizing antibodies in the reaction mixture. Preferably, the nanobodies are labeled with a detectable label which detects binding to immobilized RBD, so that, e.g. a decrease of the signal is indicative of the presence of neutralizing antibodies in the biological sample.

Alternatively, the assay can be performed detecting the binding of human antibodies from the sample to immobilized antigen/s using labeled anti-human-IgGs . By adding the novel RBD specific nanobodies of the invention to the reaction mixture, the antibodies get displaced form the antigen/s, thereby reducing the detectable signal with increasing nanobody concentration.

Thus, according to another preferred embodiment the method of the invention for screening of neutralizing SARS-CoV-2-antibodies in a biological sample can represent a competition comprising the following steps: (i) bringing into contact, in vitro, polypeptides representing SARS-CoV-2-antigen/s, e.g. the receptor binding domain (RBD) of the homotrimeric Spike protein of the SARS-CoV-2, the S1 domain of the homotrimeric Spike protein of the SARS-CoV-2 and/or the homotrimeric Spike protein of the SARS-CoV-2 with (a) a biological sample containing or suspected to contain neutralizing antibodies directed against SARS-CoV-2, (b) with anti-IgG-antibodies, and with (c) a nanobody as disclosed herein, alone or in combination with at least one other nanobody as disclosed herein, preferably in different concentrations/dilutions, to generate a reaction mixture, and (ii) detecting neutralizing SARS-CoV-2-antibodies, that are possibly present in the biological sample, which are bound to the SARS-CoV-2-antigens in the reaction mixture.

According to this embodiment, the anti-IgG-antibodies are labeled and react with a label of SARS-CoV-2-antigen/s the matching the label of the anti-IgG-antibodies, which labels, together, produce the signal.

As already mentioned above, the biological sample is selected from the group consisting of a tissue sample, body fluid sample, preferably a sputum sample and saliva sample, blood sample, preferably a whole blood sample, plasma sample or serum sample. Also, the biological sample can be a sample that has been retrieved from the mouth or pharynx of an individual by using a mouth wash or rinsing solution or similar, or by bronchial lavage.

The present invention also relates to a nucleic acid encoding an amino acid sequence comprising framework region 1, CDR1, framework region 2, CDR2, framework region 3, CDR3 and framework region 4, of the nanobody as described herein, as well as a polypeptide comprising at least one nanobody as described herein.

By providing the amino acid sequence of the nanobody of the invention, one skilled in the art is capable of producing the nanobodies according to the invention and described herein, by conventional techniques for producing polypeptides. For example, they may be synthesized by using a well known synthesis method in a solid phase (Merrifield (19962) Proc. Soc. Ex. Boil. 21:412; Merrifield (1963) J. Am. Chem. Soc. 85:2149; Tam et al. (1983) J. Am. Chem. Soc. 105:6442), preferably by using a commercial available peptide synthesis apparatus, (such as the one made by Applied Biosystems, Foster City, Calif.) and by following the instructions of the manufacturer.

Alternatively, the nanobodies according to the invention may be synthesized with recombinant DNA techniques well known one skilled in the art (Maniatis et al. (1982) Molecular Cloning: a laboratory manual, Cold Spring Harbor Laboratories, NY, 51-54 and 412-430). For example, they may be obtained as DNA expression products after incorporating DNA sequences coding for the polypeptide of interest in expression vectors and introducing these vectors in suitable prokaryotic or eukaryotic hosts which will express the polypeptide of interest, from which they may then be isolated by using techniques well known to one skilled in the art. Accordingly, the invention also concerns a vector comprising a nucleic acid coding for the polypeptides and nanobodies as disclosed herein, as well as a transfected, infected or transformed cell with such a nucleic acid or vector.

Further advantages follow from the description of the embodiments and the attached drawings.

It goes without saying that the abovementioned features and the features which are still to be explained below can be used not only in the respectively specified combinations, but also in other combinations or on their own, without departing from the scope of the present invention.

Several embodiments of the invention are illustrated in the figures and explained in more detail in the following description.

### BRIEF DESCRIPTION OF THE DRAWINGS

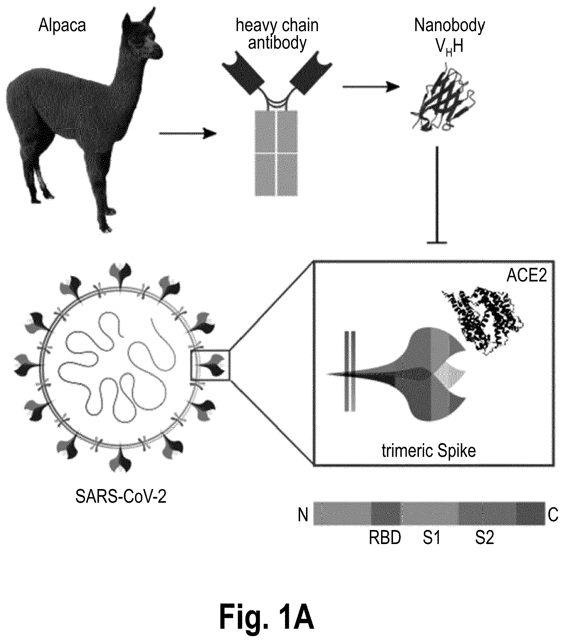

**Fig. 1** shows a schematic depiction of the generation of Nbs blocking the SARS-CoV-2 RBD:ACE2 interaction site Nanobodies (Nbs) are genetically engineered from heavy chain only antibodies of alpacas. The interaction between the SARS-CoV-2 homotrimeric Spike protein and ACE2 can be blocked by RBD-specific Nbs. Protein structures adapted from PDB 3OGO (Nb) and 6CS2 (ACE2) (**A**).; in (**B**), the sequences of 10 exemplary nanobodies are shown in sequence alignment, with "FR" designation the frame regions and "CDR" designating the regions for determining complementarity;

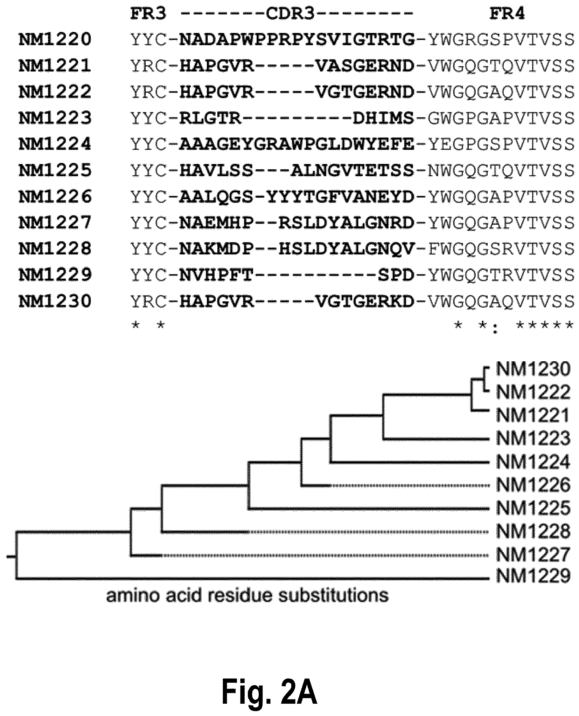

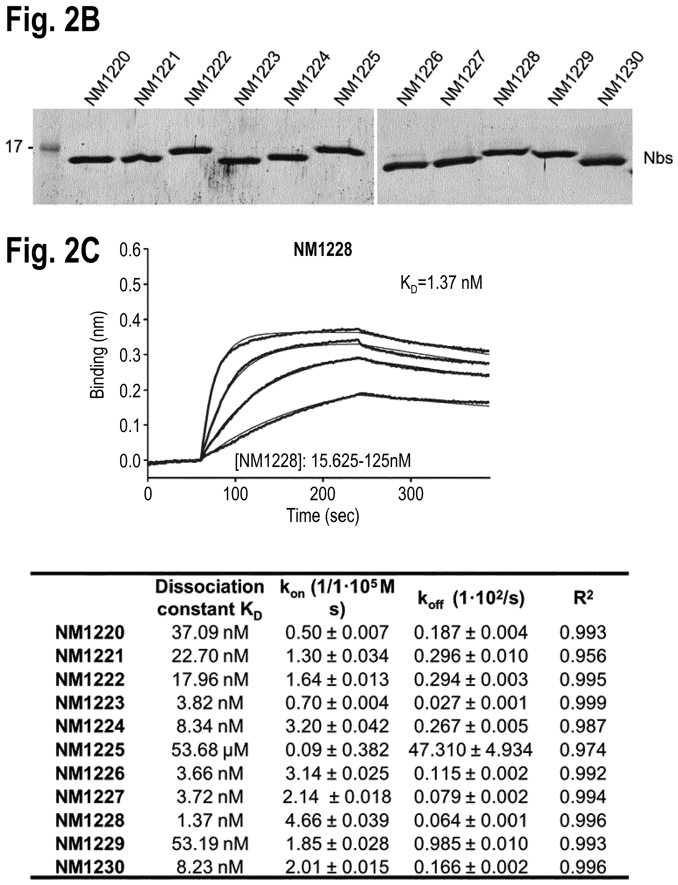

**Fig. 2** shows the results of experiments for the selection of nanobodies (Nbs) against RBD of SARS-CoV-2: (**A**) Amino acid sequences of the complementarity determining region (CDR) 3 from unique Nbs selected after two rounds of biopanning are listed. Phylogenetic tree analysis of the CDR3s or selected Nbs is shown based on a ClustalW alignment (lower panel). **B**) Recombinant expression and purification of Nbs using immobilized metal affinity chromatography (IMAC) and size exclusion chromatography (SEC). Coomassie staining of 2 µg of purified Nbs is shown (**C**) For biolayer interferometry-based affinity measurements, biotinylated RBD was immobilized on streptavidin biosensors. Kinetic measurements were performed by using four concentrations of purified Nbs ranging from 15.6 nM - 2 µM. As an example the sensogram of the NM1228 nanobody at indicted concentrations is shown. The table summarizes affinities (*KD*), association (Kon) and dissociation constants (Koff) determined for individual Nbs (lower panel).

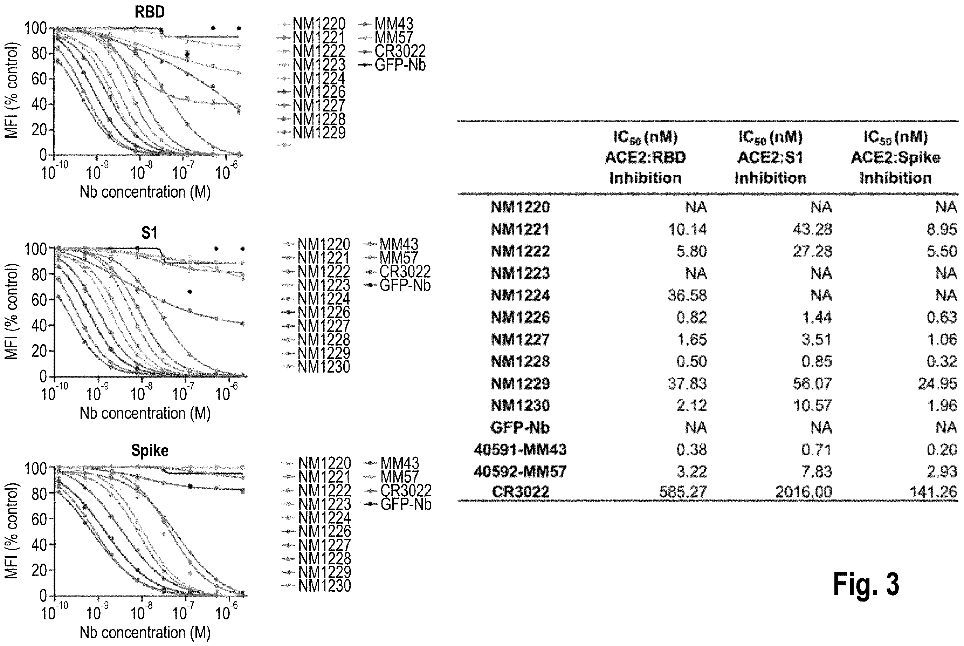

**Fig. 3** shows the results of multiplex binding assays to identify inhibitory nanobodies: Results from bead-based multiplex ACE2 competition assay are shown for the three SARS-CoV-2 Spike derived antigens, RBD, S1 and homotrimeric Spike. In the assay, ACE2 was detected bound to the respective antigen. For each nanobody, a dilution series over eight steps (2.106 µM to 0.123 nM) is shown in the presence of 80 ng/mL ACE2. MFI signals were normalized to the maximal signal per antigen as given by the ACE2-only control. IC₅₀ values were calculated from a four-parametric sigmoidal model and are displayed for each Nb and antigen. Data are presented as mean +/- stds of three technical replicates (n =3).

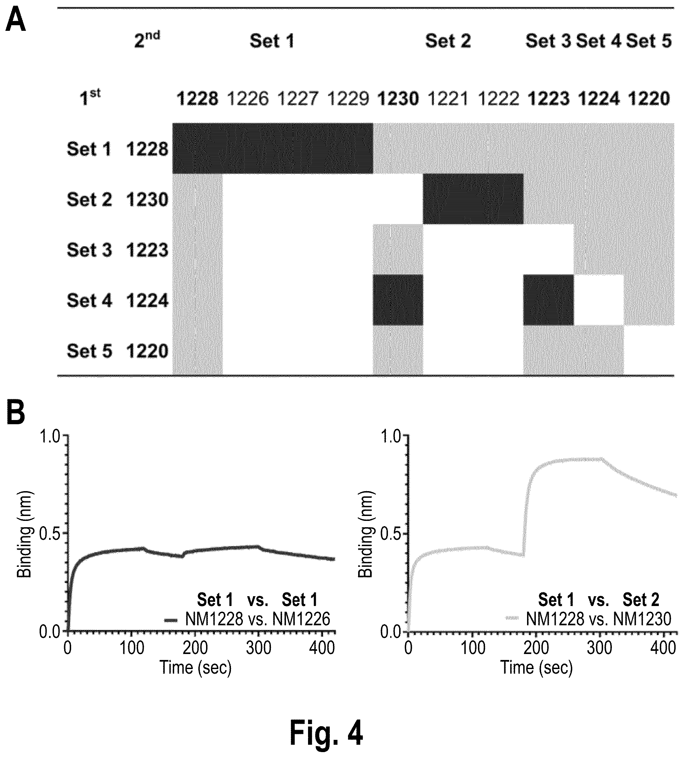

**Fig. 4** shows the results of epitope binning of nanobodies determined by Biolayer interferometry on RBD: (**A**) Heat map illustration of competitive Nb epitope binning on RBD using biolayer interferometry (BLI). Rows and columns represent the loading of the first and second Nb, respectively. Darker colored squares illustrate no additional binding of the second Nb meaning both Nbs belong to the same Nb-Set. Lighter colored squares represent additional binding of the second Nb, hence these Nbs belong to different Nb-Sets. (**B**) Representative sensograms of single BLI measurements of Nbs affiliated to the same Nb-Set (NM1228/NM1226, dark) and to different Nb-Sets (NM1228/NM1230, light) are shown.

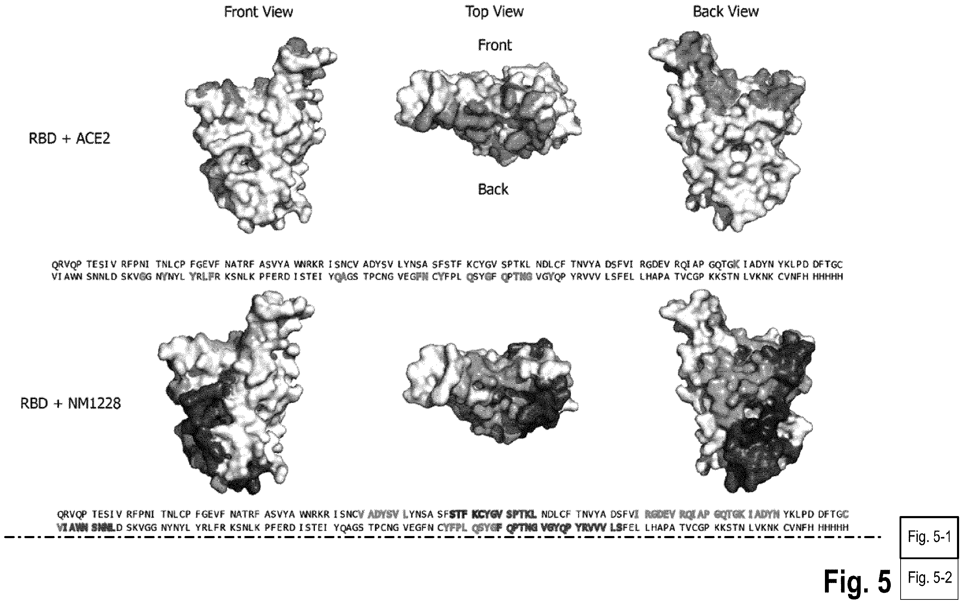

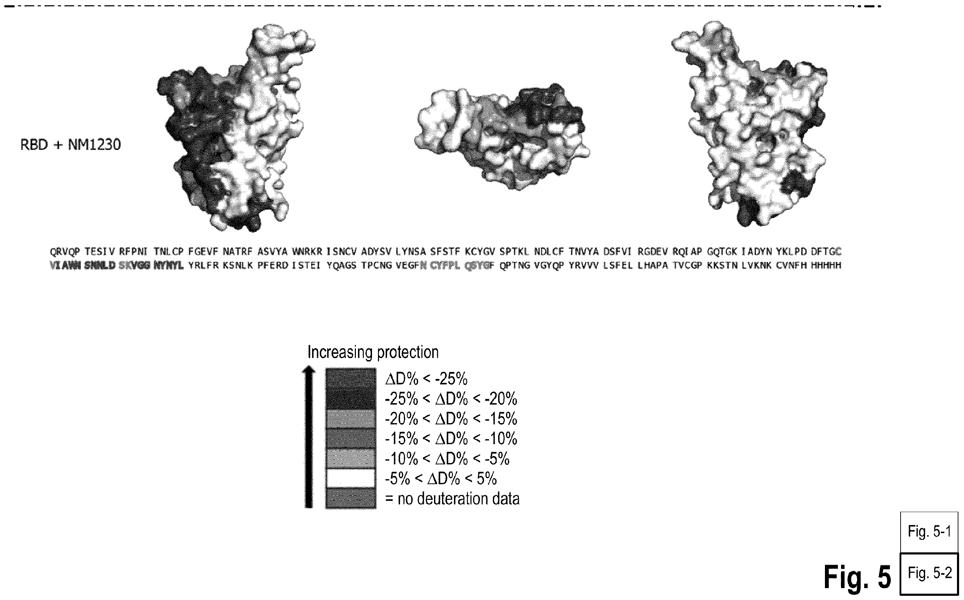

**Fig. 5** shows results of epitope mapping of nanobodies by HDX mass spectrometry: The figure shows a surface structure model of the RBD showing the ACE2 interface and the HDX-MS epitope mapping results of the various Nbs. Amino acid residues of RBD involved in the RBD:ACE2 interaction site are shown in bold (top panel). RBD epitopes protected upon Nb binding are highlighted in different shadings indicating the strength of protection. Amino acid residues which are part of the Nb epitopes are highlighted in the RBD amino sequence.

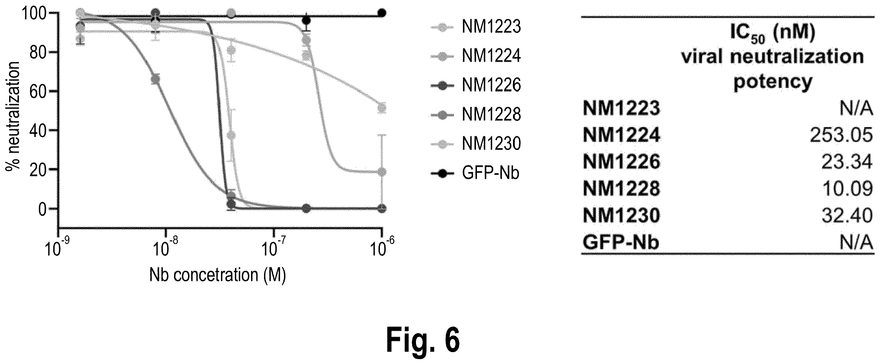

**Fig. 6** shows the results of experiments testing the viral neutralization potency of selected nanobodies: Inhibition of viral infectivity of the SARS-CoV-2 strain icSARS-CoV-2-mNG was analyzed in Caco-2 cells using serial dilutions of NM1223, NM1224, NM1226, NM1228 and NM1230. As negative control GFP-Nb was used. 48 h post-infection neutralization potency was visualized via Hoechst staining and mNeonGreen expression. Intensities of mNeonGreen signal normalized to virus-only infection control are illustrated as percent of neutralization. IC₅₀ values were calculated from a four-parametric sigmoidal model and are displayed for each Nb. Data are presented as mean +/- stds of three technical replicates (n =3).

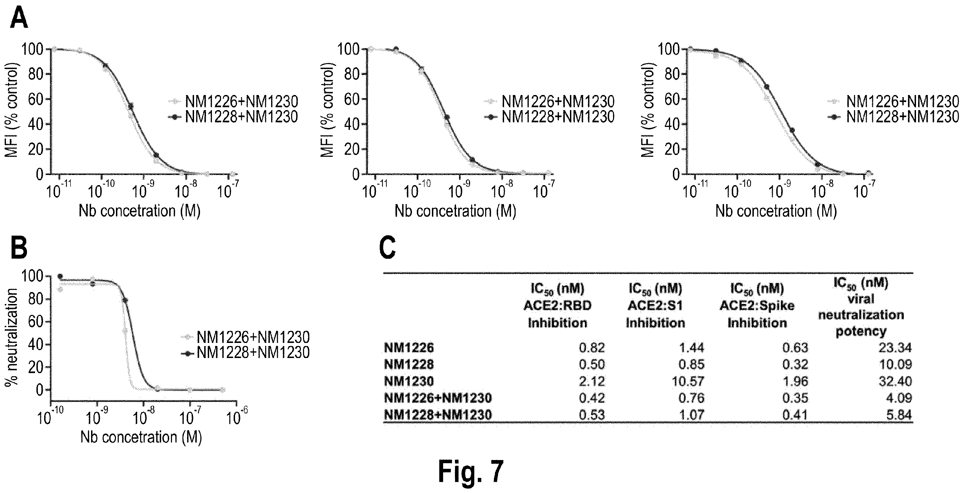

**Fig. 7** shows results of the combinatorial application of RBD nanobody-Sets for inhibition of ACE2 binding and viral neutralization: (**A**) Results from multiplex ACE2 competition assay are shown for the three Spike-derived antigens: RBD, S1 and homotrimeric Spike. Nb combinations were diluted from 126 nM to 7.69 pM per Nb in the presence of 80 ng/mL ACE2 and antigen-bound ACE2 was measured. MFI signals were normalized to the maximum detectable signal per antigen given by the ACE2-only control. IC₅₀ values were calculated from a four-parametric sigmoidal model. Data are presented as mean +/- stds of three technical replicates (n =3). (**B**) Neutralization potency of Nb-Set1 (NM1226, NM1228) in combination with Nb-Set2 (NM1230) was analyzed in Caco-2 cells using the SARS-CoV-2 strain icSARS-CoV-2-mNG. 48 h post-infection neutralization potency was visualized via Hoechst staining and mNeonGreen expression. Intensities of mNeonGreen signal normalized to virus-only infection control are illustrated as percent of neutralization. IC₅₀ values were calculated from a four-parametric sigmoidal model and are displayed for each Nb. Data are presented as mean +/- stds of three technical replicates (n =3). (**C**) Table summarizing the IC₅₀ values obtained for the individual Nbs (as shown in **Figure 3** and **Figure 6**) and the Nb combinations.

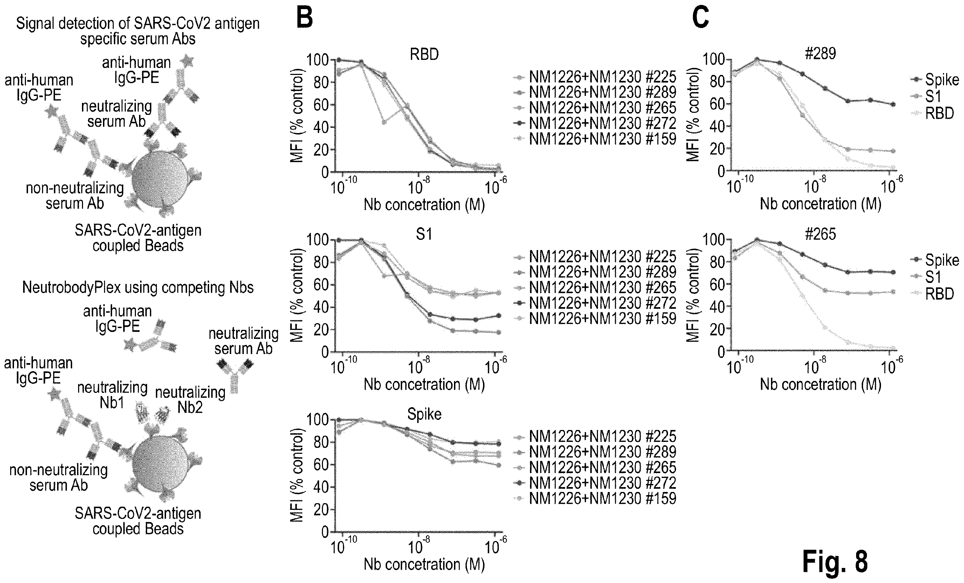

**Fig. 8** shows the results of multiplex competitive binding assays to monitor a neutralizing immune response in patients ("NetrobodyPlex"): (**A**) Schematic illustration of the NeutrobodyPlex. The immunoassay measures the ability of neutralizing RBD Nbs preventing the binding of neutralizing IgGs from patient serum detected by anti-human-IgG-PE. In presence of neutralizing IgGs, the fluorescent signal from anti-human-IgG-PE, is inversely proportional to the applied Nb concentration. (**B**) For the NeutrobodyPlex assay serial dilutions (1.26 µM to 7.69 pM per Nb) of the combination NM1226/ NM1230 were incubated with five serum samples followed by detection of bound human IgGs. Shown are MFI signals obtained for all three Spike-derived antigens normalized to serum-only control. (**C**) For two serum samples (#289, #265) differences in competition efficiency between the three Spike based antigens are shown.

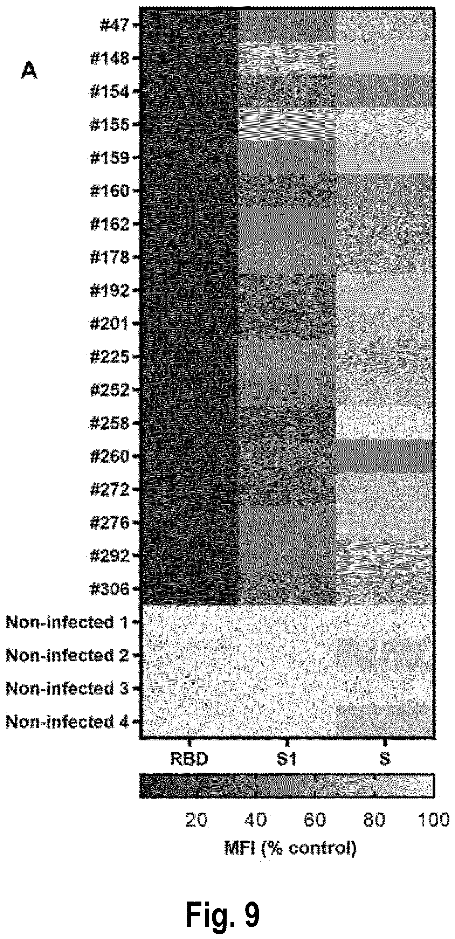

**Fig. 9** shows results of NeutrobodyPlex validation by testing of patient sample cohort in comparison to viral neutralization assay: 18 serum samples from SARS-CoV-2 convalescent patients and four from healthy donors were analyzed using the NeutrobodyPlex using a fixed concentration of the Nb combination NM1226/ NM1230 (1.26 µM per Nb). MFI values normalized to serum only control are illustrated as heat map graphic. Dark color coding represents loss of the detectable signal, meaning a strong shift of serum antibodies into the unbound state by off-competition of Nbs. Lighter color coding represents no signal differences in presence or absence of Nbs.

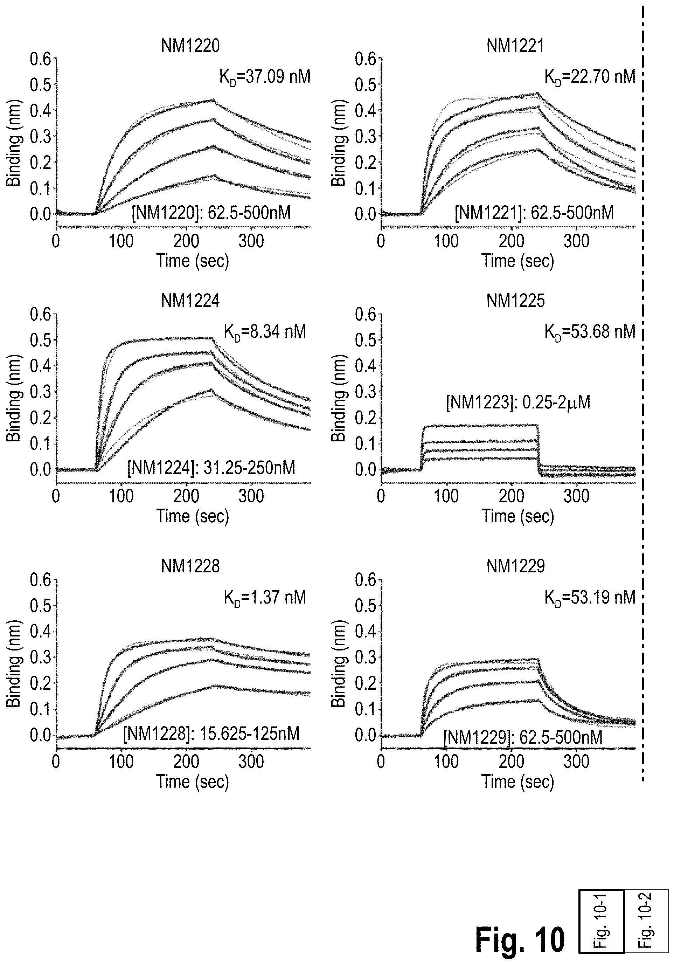

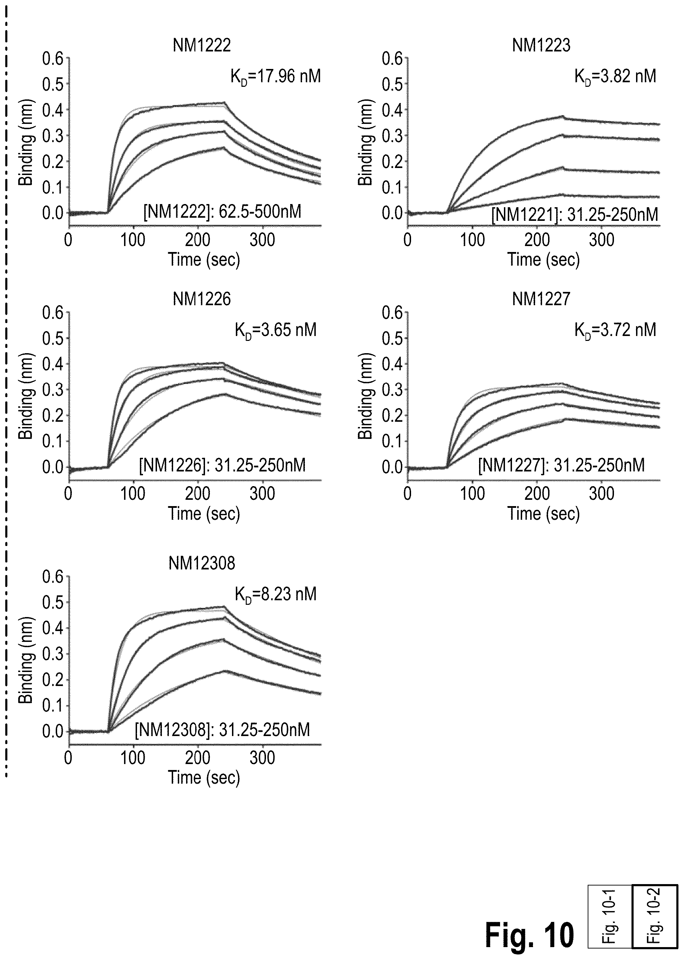

**Fig. 10** shows results of experiments for affinities of SARS-CoV-2 RBD binding Nanobodies determined by Biolayer interferometry on recombinant SARS-CoV-2 RBD: Sensograms of biolayer interferometry-based affinity measurements of 11 identified SARS-CoV-2 RBD nanobodies are shown. For analysis biotinylated RBD was immobilized on streptavidin biosensors and kinetic measurements were performed by using four concentrations of purified Nbs ranging from 15.6 nM - 2 µM.

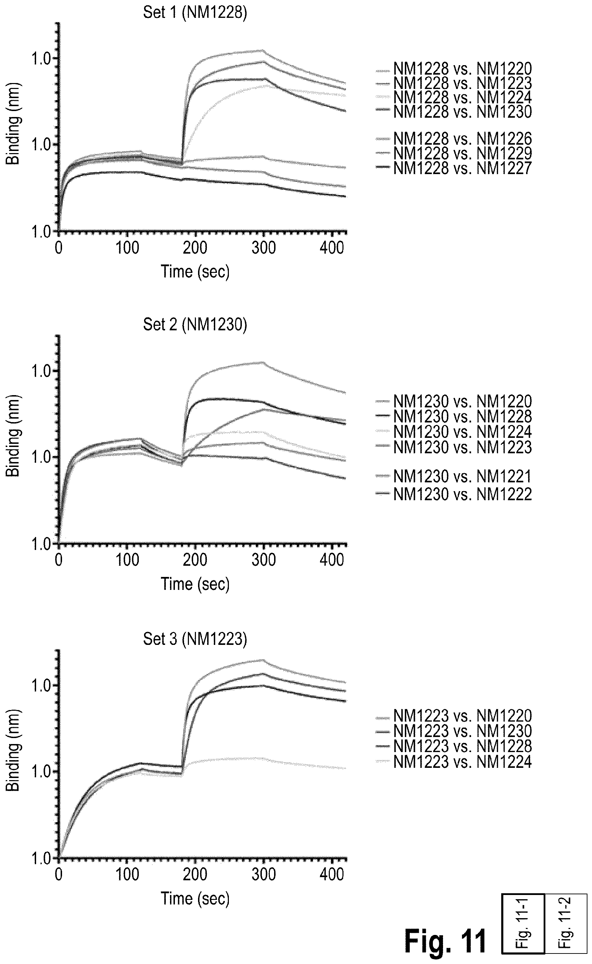

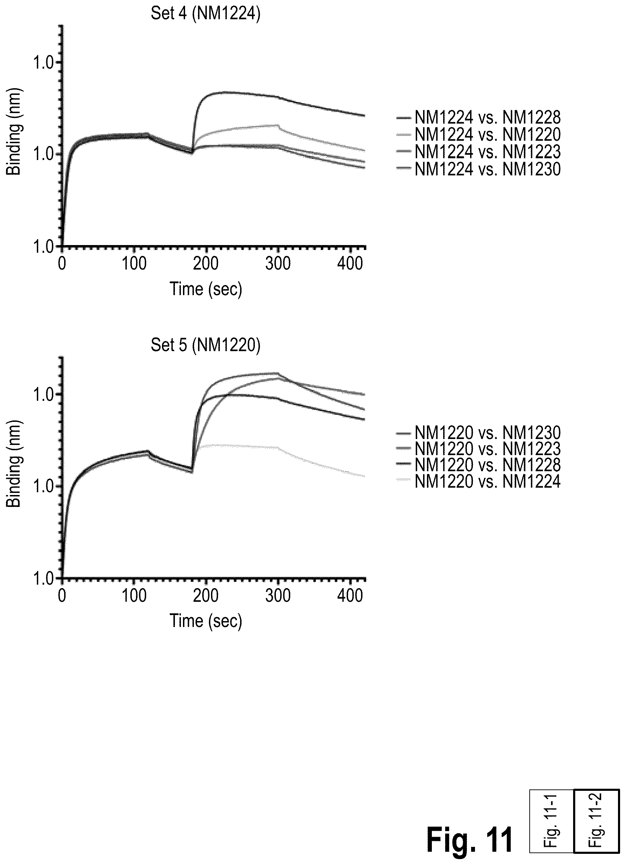

**Fig. 11** shows results of epitope binning of SARS-CoV-2 RBD Nanobodies determined by Biolayer interferometry on recombinant SARS-CoV-2 RBD: Sensograms of biolayer interferometry-based epitope binning of dual Nb binding are shown. Biotinylated RBD was immobilized on streptavidin biosensors followed by two consecutive loading steps of different RBD Nbs. Depending on additional loading (different epitope) or non-loading (similar/ overlapping epitope) of the second Nb, Nbs were clustered into different Nb-Sets. Overall, five Nb-Sets were identified. Set 1: NM1228, NM1226, NM1227, NM1229; Set 2: NM1230, NM1221, NM1222, Set 3: NM1223; Set 4: NM1224; Set 5: NM1220.

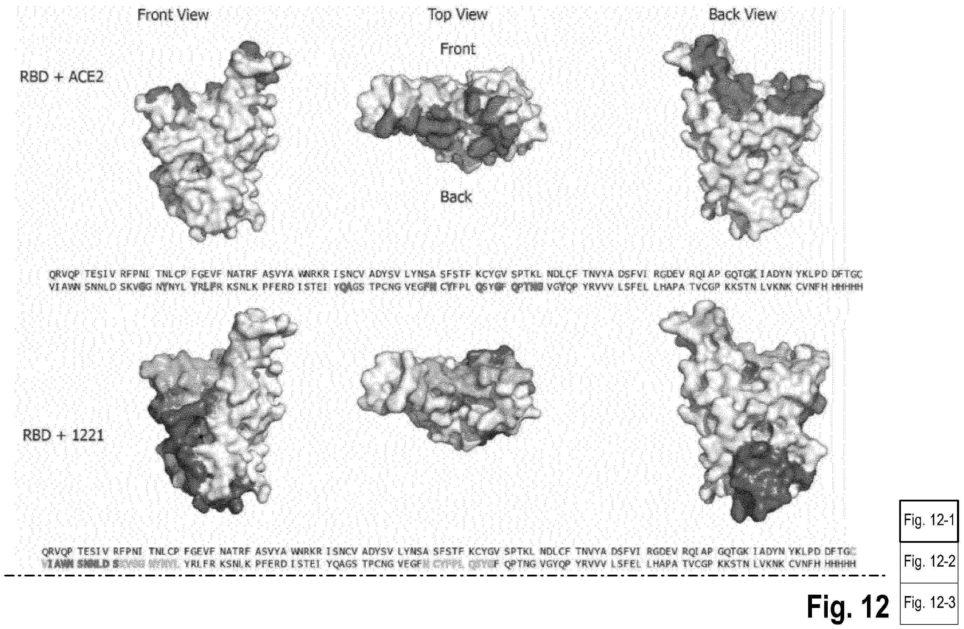

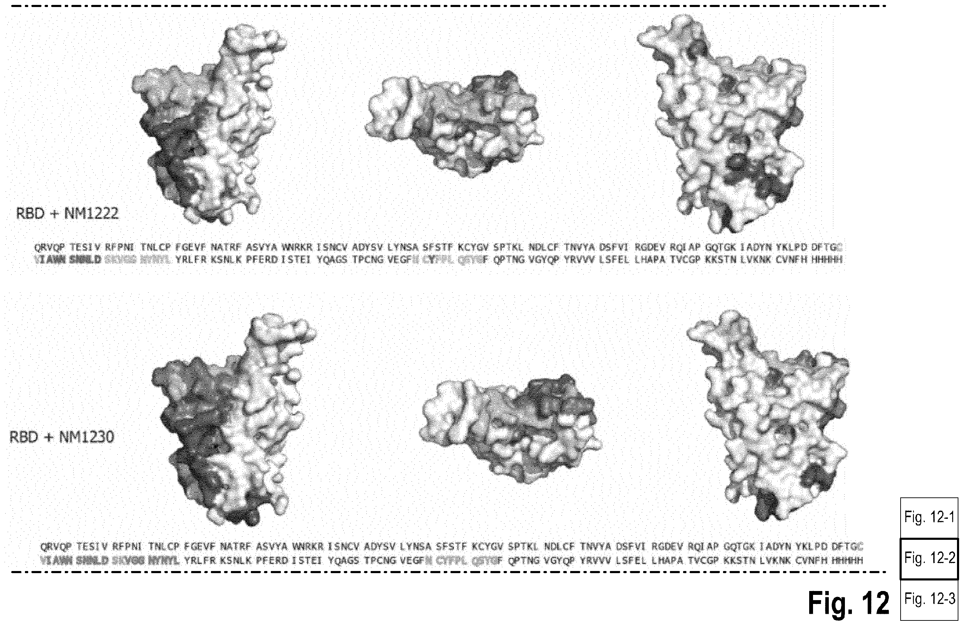

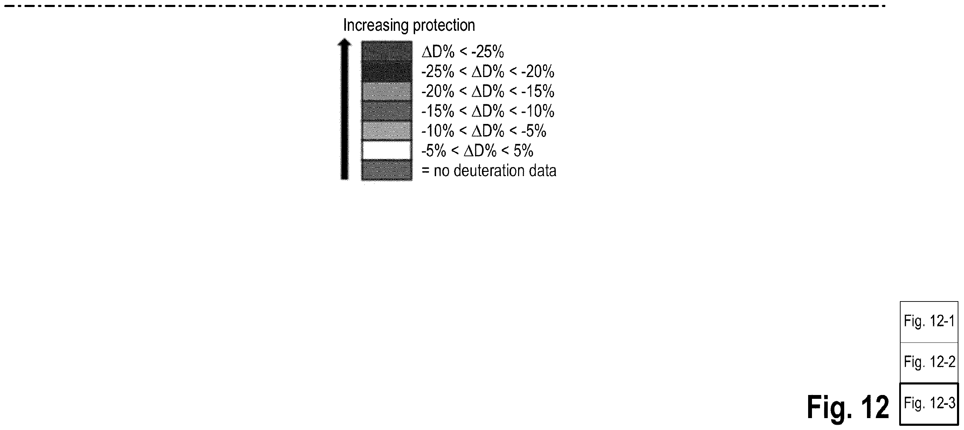

**Fig. 12** shows a surface structure model of the RBD domain showing the ACE2 interface and the HDX-MS epitope mapping results of the various nanobodies. Residues of the RBD responsible for contact of the ACE2 shown in bold (top panel). RBD epitopes protected upon binding of Nb NM1221, NM1222 and NM1230 (all chosen from Nb-Set2) are highlighted.

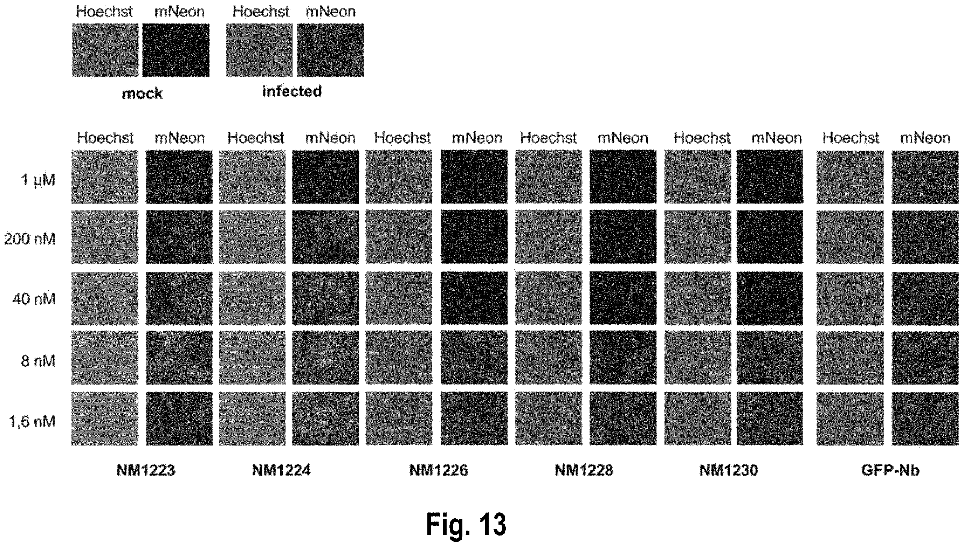

**Fig. 13** shows results for experiments about the viral neutralization potency of selected nanobodies: Inhibition of viral infectivity of the SARS-CoV-2 strain icSARS-CoV-2-mNG was analyzed in Caco-2 cells using serial dilutions of NM1223, NM1224, NM1226, NM1228 and NM1230. As negative control the GFP-Nb was used. 48 h post-infection neutralization potency was visualized via Hoechst staining and mNeonGreen expression. Representative images of human Caco-cells upon infection with SARS-CoV-2 expressing mNeonGreen (icSARS-CoV-2-mNG) either in presence or absence of serial dilutions of RBD Nbs are shown.

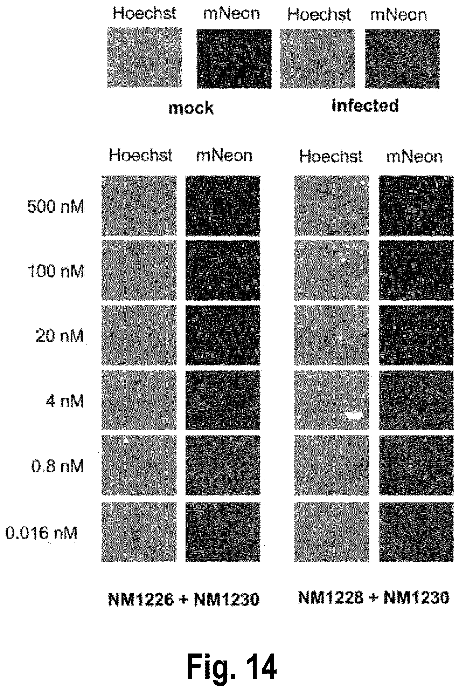

**Fig. 14** shows results for experiments about the viral neutralization with nanobodies combinations: Inhibition of viral infectivity of the SARS-CoV-2 strain icSARS-CoV-2-mNG was analyzed in Caco-2 cells using serial dilutions of Nb combinations NM1226/ NM1230 and NM1228/ NM1230. 48 h post-infection neutralization potency was visualized via Hoechst staining and mNeonGreen expression. Representative images of human Caco-cells upon infection with SARS-CoV-2 expressing mNeonGreen (icSARS-CoV-2-mNG) either in presence or absence of serial dilutions of combinations of RBD Nbs are shown.

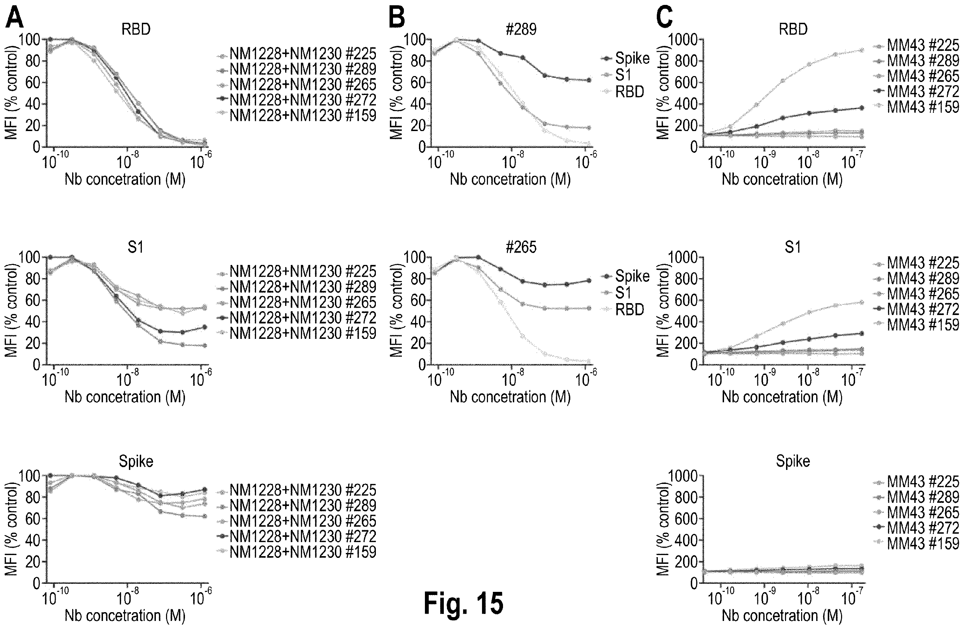

**Fig. 15** shows results of a multiplex competitive binding assay to monitor a neutralizing immune response in patients ("the NeutrobodyPlex"): (**A**) For the NeutrobodyPlex assay serial dilutions (1.26 µM to 7.69 pM per Nb) of the combination NM1228/ NM1230 were incubated with five serum samples followed by detection of bound human IgGs. Shown are MFI signals obtained for all three Spike-derived antigens normalized to serum-only control. (**B**) For two serum samples (#289, #265) differences in Nb competition efficiency between the three Spike based antigens are shown. (**C**) Curves as presented, show normalized MFI signals derived from a similar assay using the neutralizing mouse antibody MM43 in concentrations ranging from of 0.17 µM to 0.08 nM instead of Nb combination; and

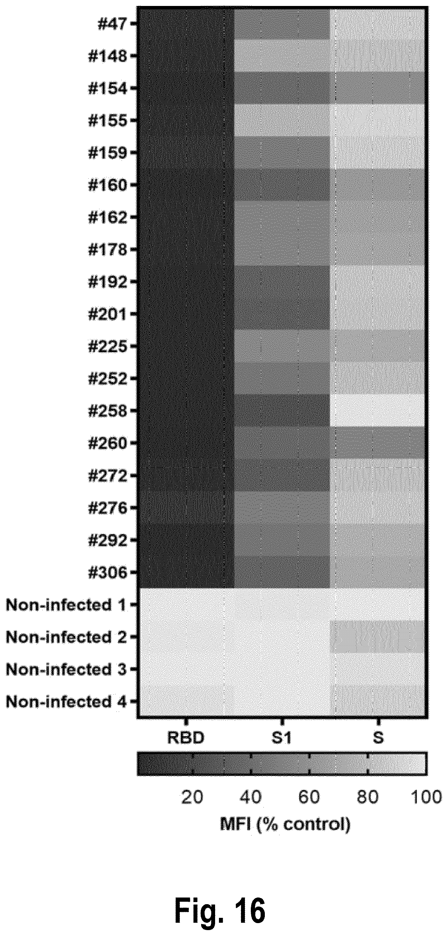

**Fig. 16** shows results of NeutrobodyPlex validation by testing of patient sample cohort: 18 serum samples from SARS-CoV-2 convalescent patients and four from healthy donors were analyzed using the NeutrobodyPlex using a fixed concentration of the Nb combination NM1228/ NM1230 (1.26 µM per Nb). MFI values normalized to serum only control are illustrated as heat map graphic. Dark color coding represents loss of the detectable signal, meaning a strong shift of serum antibodies into the unbound state by off-competition of Nbs. Lighter color coding represents no signal differences in presence or absence of Nbs.

### DETAILED DESCRIPTION OF PREFERRED EMBODIMENTS

Herein, the selection of 10 unique nanobodies (Nbs), from a nanobody gene library derived from an Alpaca immunized with glycosylated SARS-CoV-2 RBD is described. All Nbs can be readily produced in bacteria at high yields and bind their target structure with strong affinities in their monovalent format. The Nbs effectively block the interaction between RBD, S1-domain and the trimeric spike protein with ACE2 as the viral docking site in a multiplex in vitro binding assay. Based on competitive binding analysis and detailed epitope mapping using Hydrogen Deuterium Exchange mass spectrometry (HDX-MS) all ACE2 blocking Nbs were clustered in three distinct Nb-Sets and their potential to neutralize SARS-CoV-2 infection in a human cell model as demonstrated. By testing combinations from different Nb-Sets in both functional assays, substantially improved IC₅₀ values were achieved indicating a highly synergistic effect of Nbs targeting simultaneously different epitopes within the RBD. Finally, a competitive binding assay was performed using serum samples from infected individuals and the most potent inhibitory Nb combination. With this approach the presence of antibodies in the patient samples was demonstrated addressing the RBD-ACE2 interface which are in accordance to previous findings designed as being neutralizing. Based on the data presented here, the Nbs presented herein have a high potential for prophylactic and therapeutic options and provide a novel approach to screen for a neutralizing immune response in infected or vaccinated individuals thus helping to monitor the immune status or to guide vaccine design.

### Materials and Methods

**Expression constructs** For bacterial expression of V_{H}H domains (nanobodies, Nbs), sequences were cloned into the pHEN6 vector (Arbabi Ghahroudi, M., et al., "Selection and identification of single domain antibody fragments from camel heavy-chain antibodies" FEBS Lett (1997), 414(3):521-6.), thereby adding a C-terminal 6xHis-tag for IMAC purification as described previously (Kirchhofer, A., et al., "Modulation of protein properties in living cells using nanobodies" Nat Struct Mol Biol (2010), 17(1):133-8; Rothbauer, U., et al., "A versatile nanotrap for biochemical and functional studies with fluorescent fusion proteins", Mol Cell Proteomics (2008) 7(2):282-9). The pCAGGS plasmids encoding the stabilized trimeric Spike protein and the receptor binding domain (RBD) of SARS-CoV-2 were kindly provided by F. Krammer (Amanat, F., et al., "A serological assay to detect SARS-CoV-2 seroconversion in humans". Nat Med, (2020) 26(7):1033-1036). The cDNA encoding the S1 domain (aa 1 - 681) of the SARS-CoV-2 Spike protein was obtained by PCR amplification using the forward primer S1_CoV2-for 5'- CTT CTG GCG TGT GAC CGG - 3' (SEQ ID NO. and) reverse primer S1_CoV2-rev 5' - GTT GCG GCC GCT TAG TGG TGG TGG TGG TGG TGG GGG CTG TTT GTC TGT GTC TG - 3' (SEQ ID NO.) and the full length SARS-CoV-2 SPIKE cDNA as template and cloned into the Xbal/Notl-digested backbone of the pCAGGS vector, thereby adding a C-terminal His₆-Tag. All expression constructs were verified by sequence analysis.

**V_{H}H libraries** Alpaca immunizations with purified RBD and V_{H}H-library construction were carried out as described previously (Rothbauer, U., et al., "Targeting and tracing antigens in live cells with fluorescent nanobodies". Nat Methods, (2006) 3(11):887-9). Animal immunization has been approved by the government of Upper Bavaria (Permit number: 55.2-1-54-2532.0-80-14). In brief, nine weeks after immunization of an animal (*Vicugna pacos*) with either C-terminal histidine-tagged RBD (RBD-His₆), ∼100 ml blood were collected and lymphocytes were isolated by Ficoll gradient centrifugation using the Lymphocyte Separation Medium (PAA Laboratories GmbH). Total RNA was extracted using TRIzol (Life Technologies) and mRNA was reverse transcribed to cDNA using a First-Strand cDNA Synthesis Kit (GE Healthcare). The V_{H}H repertoire was isolated in 3 subsequent PCR reactions using following primer combinations (1) CALL001 (5'-GTC CTG GCT GCT CTT CTA CA A GG-3') (SEQ ID NO: ) and CALL002 (5'-GGT ACG TGC TGT TGA ACT GTT CC-3') (SEQ ID NO:) (2) forward primer set FR1-1, FR1-2, FR1-3, FR1-4 (5'-CAT GGC NSA NGT GCA GCT GGT GGA NTC NGG NGG-3' (SEQ ID NO:), 5'-CAT GGC NSA NGT GCA GCT GCA GGA NTC NGG NGG-3' (SEQ ID NO:), 5'-CAT GGC NSA NGT GCA GCT GGT GGA NAG YGG NGG-3' (SEQ ID NO:), 5'-CAT GGC NSA NGT GCA GCT GCA GGA NAG YGG NGG-3' (SEQ ID NO:)) and reverse primer CALL002 and (3) forward primer FR1-ext1 and FR1-ext2 (5'-GTA GGC CCA GCC GGC CAT GGC NSA NGT GCA GCT GGT GG-3' (SEQ ID NO:), 5'-GTA GGC CCA GCC GGC CAT GGC NSA NGT GCA GCT GCA GGA-3' (SEQ ID NO:) A-) and reverse primer set FR4-1, FR4-2, FR4-3, FR4-4, FR4-5 and FR4-6 (5'-GAT GCG GCC GCN GAN GAN ACG GTG ACC NGN RYN CC-3'(SEQ ID NO:). 5'-GAT GCG GCC GCN GAN GAN ACG GTG ACC NGN GAN CC-3' (SEQ ID NO:). 5'-GAT GCG GCC GCN GAN GAN ACG GTG ACC NGR CTN CC-3' (SEQ ID NO:). 5'-GAT GCG GCC GCR CTN GAN ACG GTG ACC NGN RYN CC-3' (SEQ ID NO:). 5'-GAT GCG GCC GCR CTN GAN ACG GTG ACC NGN GAN CC-3' (SEQ ID NO:). 5'-GAT GCG GCC GCR CTN GAN ACG GTG ACC NGR CTN CC-3' (SEQ ID NO:)) (introducing Sfil and Notl restriction sites. The V_{H}H library was subcloned into the Sfil/Notl sites of the pHEN4 phagemid vector (Arbabi Ghahroudi, M., et al., "Selection and identification of single domain antibody fragments from camel heavy-chain antibodies" FEBS Lett, (1997) 414(3):521-6).

**V_{H}H Screening** For the selection of RBD-specific V_{H}Hs two consecutive phage enrichment rounds were performed. Therefore, TG1 cells containing the 'immune'-library in pHen4 were infected with the M13K07 helper phage, hence the V_{H}H domains were presented superficial on phages. For each round 1 x 10¹¹ phages of the 'immune'-library were applied on RBD either directly coated on immunotubes (10 µg/ml) or biotinylated RBD (5 µg/ml) immobilized on 96-well plates pre-coated with Streptavidin. In each selection round extensive blocking of antigen and phages was performed by using 5% milk or BSA in PBS-T and with increasing panning round PBS-T washing stringency was intensified. Bound phages were eluted in 100 mM tri-ethylamind, TEA (pH 10.0), followed by immediate neutralization with 1 M Tris/HCI (pH 7.4). For phage preparation for following rounds, exponentially growing TG1 cells were infected and spread on selection plates. Antigen-specific enrichment for each round was monitored by comparing colony number of antigen vs. no antigen selection. Following panning 492 individual clones of the second selection round were screened by standard Phage-ELISA procedures using a horseradish peroxidase-labeled anti-M13 monoclonal antibody (GE-Healthcare).

**Protein expression and purification.** RBD-specific Nbs were expressed and purified as previously published (Kirchhofer, A., et al., "Modulation of protein properties in living cells using nanobodies" Nat Struct Mol Biol (2010), 17(1):133-8; Rothbauer, U., et al., "A versatile nanotrap for biochemical and functional studies with fluorescent fusion proteins", Mol Cell Proteomics (2008) 7(2):282-9). For the expression of SARS-CoV-2 proteins (RBD, stabilized homotrimeric Spike and S1 domain) Expi293 cells were applied according to the protocol of Stadlbauer *et al.* (Stadlbauer, D., et al., "SARS-CoV-2 Seroconversion in Humans: A Detailed Protocol for a Serological Assay, Antigen Production, and Test Setup"Curr Protoc Microbiol, (2020) 57(1):e100). For quality control all purified proteins were analyzed via SDS-PAGE according to standard procedures. Therefore, protein samples were denaturized (5 min, 95°C) in 2x SDS-sample buffer containing 60 mM Tris/HCI, pH 6.8; 2% (w/v) SDS; 5% (v/v) 2-mercaptoethanol, 10% (v/v) glycerol, 0.02% bromphenole blue. All proteins were visualized by InstantBlue Coomassie (Expedeon) staining. For immunoblotting proteins were transferred on nitrocellulose membrane (Bio-Rad Laboratories) and detection was performed using anti-His primary antibody (Penta-His Antibody, #34660, Qiagen) followed by donkey-anti-mouse secondary antibody labeled with AlexaFluor647 (Invitrogen) using a Typhoon Trio scanner (GE-Healthcare, Freiburg, Germany; excitation 633 nm, emission filter settings 670 nm BP 30).

**Biophysical Biolayer interferometry (BLI)** For analyzing the binding affinity of purified Nbs towards RBD biolayer interferometry (BLItz, ForteBio) was performed. Therefore, biotinylated RBD was immobilized on single-use high-precision streptavidin biosensors (SAX) according to manufacturer's protocols. Depending on the affinity of the RBD-Nb interaction, an appropriate concentration range (15.6 nM-2 µM) of Nbs were used. In total for each run four different Nb concentrations were measured as well as a reference run using PBS instead of Nb in the association step. As negative control the GFP-Nb (500nM) was applied in the binding studies. By this means global fits were obtained using the BLItzPro software and the global dissociation constant (K_{D}) was calculated. For the epitope competition analysis biotinylated RBD was immobilized on streptavidin sensor in the same fashion as for the affinity measurements. By two consecutive application steps each with association and short dissociation of two different Nbs (500nM) competition binding was performed.

**Bead-based multiplex binding/competition assay** Purified RBD, S1 domain and homotrimeric Spike of SARS-CoV-2 were covalently immobilized on spectrally distinct populations of carboxylated paramagnetic beads (MagPlex Microspheres, Luminex Corporation, Austin, TX) using 1-ethyl-3-(3-dimethylaminopropyl)carbodiimide (EDC)/ sulfo-N-hydroxysuccinimide (sNHS) chemistry. For immobilization, a magnetic particle processor (KingFisher 96, Thermo Scientific, Schwerte, Germany) was used. Bead stocks were vortexed thoroughly and sonicated for 15 seconds. Subsequently, 83 µL of 0.065% (v/v) Triton X-100 and 1 mL of bead stock containing 12.5 x 10⁷ beads of one single bead population were pipetted into each well. The beads were then washed twice with 500 µL of activation buffer (100 mM Na₂HPO₄, pH 6.2, 0.005% (v/v) Triton X-100) and activated for 20 min in 300 µL of activation mix containing 5 mg/mL EDC and 5 mg/mL sNHS in activation buffer. Following activation, the beads were washed twice with 500 µL of coupling buffer (500 mM MES, pH 5.0, 0.005% (v/v) Triton X-100) and the proteins were added to the activated beads and incubated for 2 h at 21 °C to immobilize the antigens on the surface. Protein-coupled beads were washed twice with 800 µL of wash buffer (1x PBS, 0.005 % (v/v) Triton X-100) and were finally resuspended in 1,000 µL of storage buffer (1x PBS, 1 % (w/v) BSA, 0.05% (v/v) ProClin). The beads were stored at 4°C until further use. For bead-based multiplex assays, individual bead populations were combined into a bead mix.

For the bead-based ACE2 competition binding assay, the nanobodies were incubated with the bead-mix containing beads coupled with SARS-CoV-2 homotrimeric Spike, RBD and S1 proteins in the presence of biotinylated ACE2 (Sino Biological) competing for the binding of SARS-CoV-2 spike-derived antigens. Single nanobodies or nanobody combinations were pre-diluted to a concentration of 6.3 µmol/L per nanobody in assay buffer. Afterwards, a 4-fold dilution series was made over eight steps in assay buffer containing 160 ng/mL biotinylated ACE2. Subsequently, 25 µL of every dilution was transferred to 25 µL bead-mix in a 96-well half-area plate. The plate was incubated for 2 h at 21 °C, shaking at 750 rpm. Afterwards, the beads were washed using a microplate washer (Biotek 405TS, Biotek Instruments GmbH) to remove unbound ACE2 or nanobodies. The beads were then incubated with R-phycoerythrin-labeled streptavidin to detect biotinylated ACE2 that bound to the immobilized antigen for 45 minutes at 21 °C shaking at 750 rpm. Afterwards, the beads were washed again to remove unbound PE-labeled streptavidin. Measurements were performed with a FLEXMAP 3D instrument using the xPONENT Software version 4.3 (settings: sample size: 80 µL, 50 events, Gate: 7,500 - 15,000, Reporter Gain: Standard PMT).

**Bead-based multiplex neutralizing antibody detection assay** A bead-mix containing beads coupled with purified RBD, S1 domain and homotrimeric Spike of SARS-CoV-2 was used. For the neutralizing antibody detection assay two nanobody combinations were used with five serum samples. The combinations were either incubated at different concentrations over 4-fold dilution series ranging from 1.26 µM to 0.08 nmol/L for each nanobody together with a 400-fold diluted serum sample or as a negative control without any serum. For a positive control, serum as was incubated in a 400-fold dilution. As negative control for nanobody binding a SARS-CoV-2-unspecific GFP nanobody (1.26 µM) was used. As a respective positive control an inhibiting mouse antibody (40591-MM43) was applied in a concentration of 0.17 µM to 0.08 nM.

### Hydrogen-Deuterium Exchange

### RBD Deuteration Kinetics and Epitope Elucidation

RBD (5 µL, 73 µM) was either incubated with PBS or RBD-specific Nbs (2.5 µL, 2.5 mg/mL in PBS) at 25 °C for 10 min. Deuterium exchange of the pre-incubated nanobody-antigen complex was initiated by dilution with 67.5 µL PBS (150 mM NaCl, pH 7.4) prepared with D₂O and incubation for 5 and 50 minutes respectively at 25 °C. To ensure a minimum of 90% of complex formation, the molar ratio of antigen to Nbs was calculated according to (Kochert, B.A., et al., "Hydrogen-Deuterium Exchange Mass Spectrometry to Study Protein Complexes" Methods Mol Biol, (2018) 1764:153-171) using the affinity constants of 1.37 nM (NM1228), 3.66 nM (NM1226), 3.82 nM (NM1223), 8.23 nM (NM1230) and 8.34 nM (NM1224) determined by BLI analysis. The final D₂O concentration was 90%. After 5 and 50 min at 25 °C, aliquots of 15 µL were taken and quenched by adding 15 µL ice-cold quenching solution (0.2 M TCEP with 1.5% formic acid and 4 M guanidine HCI in 100 mM ammonium formate solution pH 2.2) resulting in a final pH of 2.5. Quenched samples were immediately snap frozen. The immobilized pepsin was prepared by adding 60 µl of 50% slurry (in ammonium formate solution pH 2.5) to a tube and dried by centrifugation at 1000 x g for 3 min at 0 °C and discarding the supernatant. Before injection, aliquots were thawed and added to the dried pepsin beads. Proteolysis was performed for 2 min in a water ice bath followed by filtration using a 22 µm filter and centrifugation at 1000 x g for 30 s at 0 °C. Samples were immediately injected to a LC-MS system. Undeuterated control samples were prepared under the same conditions using H₂O instead of D₂O. The same protocol was applied for the nanobodies without addition of RBD as well to create a list of peptic peptides. The HDX experiments of the RBD-Nb-complex were performed in triplicates. The back-exchange of the method as determined using a standard peptide mixture of 14 synthetic peptides was 24%.

### Chromatography and Mass Spectrometry

HDX samples were analyzed on a LC-MS system comprised of RSLC pumps (UltiMate 3000 RSLCnano, Thermo Fisher Scientific, Dreieich, Germany), a chilling device for chromatography (MéCour Temperature Control, Groveland, MA, USA) and a mass spectrometer Q Exactive (Thermo Fisher Scientific, Dreieich, Germany) The chilling device contained the LC column (ACQUITY BEH C18, 1.7 µm, 300 Å, 1 mm x 50 mm (Waters GmbH, Eschborn, Germany)), a cooling loop for HPLC solvents, a sample loop, and the injection valve and kept them at 0 °C. Samples were analyzed using a two-step 20 min linear gradient with a flow rate of 50 µl/min. Solvent A was 0.1% (v/v) formic acid and solvent B was 80% acetonitrile (v/v) with 0.1% formic acid (v/v). After 3 min desalting at 10% B, a 9 min linear gradient from 10 to 25% B was applied followed by an 8 min linear gradient from 25 to 68.8%. Experiments were performed using a Q Exactive (Thermo Fisher Scientific, Dreieich, Germany) with 70,000 resolutions instrument configurations as follows: sheath gas flow rate of 25; aux gas flow rate of 5; S-lens RF level of 50, spray voltage of 3.5 kV and a capillary temperature of 300 °C.

### HDX Data Analysis

A peptic peptide list containing peptide sequence, retention time and charge state was generated in a preliminary LC-MS/MS experiment. The peptides were identified by exact mass and their fragment ion spectrum using protein database searches by Proteome Discoverer v2.1.0.81 (Thermo Fisher Scientific, Dreieich, Germany) and implemented SEQUEST HT search engine. The protein database contained the RBD and the pepsin sequences. Precursor and fragments mass tolerance were set to 6 ppm and 0.05 Da, respectively. No enzyme selectivity was applied, however identified peptides were manually evaluated to exclude peptides originated through cleavage after arginine, histidine, lysine, proline and the residue after proline (Hamuro, Y. and S.J. Coales, "Optimization of Feasibility Stage for Hydrogen/Deuterium Exchange Mass Spectrometry" J Am Soc Mass Spectrom, (2018) 29(3):623-629). FDR was estimated using q-values calculated by Perculator and only peptides with high-confidence identification (q-value ≤ 0.01) were included to the list. Peptides with overlapping mass, retention time and charge in nanobody and antigen digest, were manually removed. The deuterated samples were recorded in MS mode only and the generated peptide list was imported into HDExaminer v2.5.0 (Sierra Analytics, Modesto, CA, USA). Deuterium uptake was calculated using the increase of the centroid mass of the deuterated peptides. HDX could be followed for 79% of the RBD amino acid sequence. The calculated percentage deuterium uptake of each peptide between RBD-Nb and RBD-only were compared. Any peptide with uptake reduction of 5% or greater upon Nb binding was considered as protected.

Cell culture. Caco-2 (Human Colorectal adenocarcinoma) cells were cultured at 37°C with 5% CO2 in DMEM containing 10% FCS, with 2 mM I-glutamine, 100 µg/ml penicillin-streptomycin and 1% NEAA.

**Viruses.** All experiments associated with the authentic virus were conducted in Biosafety Level 3 laboratory. The recombinant SARS-CoV-2 expressing mNeonGreen (icSARS-CoV-2-mNG) (PMID: 32289263) was obtained from the World Reference Center for Emerging Viruses and Arboviruses (WRCEVA) at the UTMB (University of Texas Medical Branch). To generate icSARS-CoV-2-mNG stocks, 200.000 Caco-2 cells were infected with 50 µl of virus in a 6-well plate, the supernatant was harvested 48 hpi, centrifuged, and stored at -80°C. For MOI determination, a titration using serial dilutions of the mNeonGreen (icSARS-CoV-2-mNG) was conducted. The number of infectious virus particles per ml was calculated as the (MOI × cell number)/(infection volume), where MOI = -In(1 - infection rate).

**Neutralization assay.** For neutralization experiments, 1 ×10⁴ Caco-2 cells/well were seeded in 96-well plates the day before infection in media containing 5% FCS. Caco-2 cells were co-incubated with the SARS-CoV-2 strain icSARS-CoV-2-mNG at a MOI=1.1 and nanobodies in serial dilutions in the indicated concentrations. 48 hpi cells were fixed with 2% PFA and stained with Hoechst33342 (1 µg/mL final concentration) for 10 minutes at 37°C. The staining solution was removed and exchanged for PBS. For quantification of infection rates, images were taken with the Cytation3 (Biotek) and Hoechst+ and mNG+ cells were automatically counted by the Gen5 Software (Biotek). Data were normalized to respective virus-only infection control. Inhibitory concentration 50 (IC₅₀) was calculated as the half-maximal inhibitory dose using 4-parameter nonlinear regression (GraphPad Prism).

**Analyses and Statistics** Graph preparation and statistical analysis was performed using the GraphPad Prism Software (Version 8.3.0).

**Patient samples** A total of 5 serum samples from SARS-CoV-2 reconvalescent donors were analyzed in the course of this study. All samples used were de-identified and pre-existing. Ethical consent was granted from the Ethics commission of the University of Tübingen under the votum 179/2020/BO2. Samples were classified as SARS-CoV-2 infected, due to a self-reported positive SARS-CoV-2 RT-PCR.

### Selection of nanobodies binding to receptor binding domain of SARS-CoV-2

To generate nanobodies directed against the receptor binding domain (RBD) of the homotrimeric Spike protein of the SARS-CoV-2 we expressed and purified SARS-CoV-2 RBD as antigen from mammalian (ExpiHEK) cells and immunized an alpaca (*Vicugna pacos*) following a 64-day immunization protocol. Subsequently a nanobody phagemid library comprising ∼ 4 x 10⁷ clones was generated representing the full repertoire of the variable heavy chains of heavy chain antibodies (VHHs or nanobodies, Nbs) derived from the animal. The library was subjected to phage display and biopanning was performed using either passively absorbed or biotinylated RBD immobilized on streptavidin plates. After two phage display cycles 492 individual clones were analyzed in a solid-phase phage ELISA and 325 positive binders were identified. Sequence analysis of 72 clones revealed 11 Nbs which cluster in eight with highly diverse complementarity determining regions (CDR) 3 (Figure 2 A). Individual Nbs were cloned with a C-terminal 6xHis-tag, expressed in *Eschericha coli* (*E.coli*) and purified using immobilized metal ion affinity chromatography (IMAC) followed by size exclusion chromatography (SEC) (Figure 2 B). For affinity measurements biolayer interferometry (BLI) was used and immobilized biotinylated RBD on the sensor tip. Incubation with serial dilutions of the Nbs revealed KD values ranging from ∼ 1.37 nM to 53.2 nM indicating a good binding of the Nbs in the monovalent format. NM1225 revealed a binding affinity in the micomolar range and was therefore not considered for further analysis. (Figure 2 C, Figure 10).

### Nbs competes with ACE2 for RBD, S1 and SPIKE binding

Next, the potential of the Nbs to block the interaction between homotrimeric Spike, S1 domain or the RBD to ACE2 was analyzed. To this end, a multiplexed binding assay was set up where the respective SARS-CoV-2 derived proteins were first covalently coupled on spectrally distinct populations of paramagnetic beads (MagPlex Microspheres) (Becker, M., et al., "Going beyond clinical routine in SARS-CoV-2 antibody testing - A multiplex corona virus antibody test for the evaluation of cross-reactivity to endemic coronavirus antigens" medRxiv, (2020): p. 2020.07.17.20156000). For parallel-ized analysis these beads were merged and simultaneously incubated with biotinylated ACE2. Binding of ACE2 to distinct viral antigens was detected on a Luminex instrument using R-phycoerythrin (PE)-labeled streptavidin (data not shown). To screen for inhibitory Nbs, linear dilutions of purified Nbs were added ranging from 12.3 pM to 2 µM in combination with 80 ng/mL ACE2 to the antigen-coated bead mix. After an incubation period followed by several washing steps residual binding of ACE2 to the immobilized antigens was measured. As negative control a non-specific Nb (GFP-Nb) and as positive control two inhibiting mouse antibodies were used (Gorshkov, K., et al., "Quantum Dot-Conjugated SARS-CoV-2 Spike Pseudo-Virions Enable Tracking of Angiotensin Converting Enzyme 2 Binding and Endocytosis"ACS Nano, (2020)). Data obtained with this multiplex binding assay showed that eight of the 10 analyzed Nbs inhibit ACE2 binding to isolated RBD, S1 domain and homotrimeric Spike. IC₅₀ values calculated for inhibition of ACE2-RBD-interaction ranges from 0.5 nM for NM1228 to 38 nM for NM1229 (Figure 3). Notably, IC₅₀ values obtained for the most potent inhibitory Nbs NM1228 (0.5 nM), NM1226 (0.85 nM) and NM1230 (2.12 nM) are highly comparable to IC₅₀ values measured for the conventional IgGs (40591-MM43: 0.38 nM; 40592-MM57: 3.22 nM). Additionally, the assay revealed that all Nbs except NM1224 show a highly similar inhibitory effect of ACE2 binding to all tested antigens. NM1224 seems to exclusively bind to RBD and does not prevent binding of ACE2 neither to homotrimeric Spike nor the S1 domain.

### Epitope binning

After identifying RBD specific Nbs showing an inhibitory effect on ACE2 binding, the distribution of the epitopes within the RBD was assessed which are addressed by the different Nbs. Thus, first epitope binning was performed using BLI. After coating the sensor with biotinylated RBD, the first Nb was loaded until binding saturation was reached, followed by a short dissociation step to remove excess Nb. In the following, the second Nb from a different family was exposed to the RBD-Nb-complex. In case the second Nb competes with the first one, no additional binding to RBD was observed, whereas a clear binding signal was visible, if the second Nb binds to RBD in a non-competitive manner with respect to the first Nb. Using this approach, Nbs were identified which recognize similar or overlapping epitopes on RBD (Figure 4, Figure 11). As expected, Nbs with only minor differences in their CDR3 (NM1221, NM1222 and NM1230, Nb-Set 2) were suggested to recognize an identical or highly similar epitope as they cannot bind simultaneously to RBD. Interestingly, the analysis revealed that Nbs with highly diverse CDR3s such as NM1228, NM1226, NM1227 and NM1229 also could not bind simultaneously, suggesting that these Nbs recognize similar or at least overlapping epitopes. Accordingly, these diverse Nbs were clustered in Nb-Set 1. In total, based on epitope binning, we clustered five distinct Nbs-Sets were clustered, comprising at least one candidate targeting a different epitope within the RBD compared to any member of a different Nb-Set (Figure 4).

### Epitope mapping of RBD binding nanobodies

Next, Hydrogen-Deuterium Exchange Mass Spectrometry (HDX-MS) was performed with the most potent inhibitory nanobodies selected from the different Nb-Sets to study where each of them contacts the surface of the RBD, and allowing comparison with the ACE2 binding site on the RBD. Due to their high affinity and inhibitory potency with NM1226 and NM1228 two candidates of Nb-Set1 were added. As control NM1223 was included, which was shown not to block the interaction between RBD and ACE2. As might be expected, all of the inhibitory Nbs contact the RBD in a manner that overlaps the RBD residues that comprise the ACE2 interface (Figure 5). NM1226 and NM1228, both selected from Nb-Set1, bind to HDX-defined region from the back or lower right side (back view, Figure 5), whereas NM1230 partly covers the spike-like loop region on the one edge of ACE2 interface and the top front or lower left side (front view, Figure 5).

Additionally, comparing the epitope binning assays with the HDX-MS results provides structural insights into the mechanism by which non-competing pairs of Nbs can simultaneously bind the RBD. Interestingly the combination of NM1228 or NM1226 (Nb-Set1) with NM1230 (Nb-Set2) shows a nearly complete coverage of the ACE2 interface (Figure 5). From these findings it is supposed that such a combination acts synergistically on the inhibition of the interaction between RBD and ACE2. RBD in combination with NM1221, NM1222 and NM1230 showed the same protected regions which is in good agreement with the binning data (Fig. 12).

### RBD nanobodies can potently neutralize the SARS-CoV-2 virus

Knowing that the RBD nanobodies inhibit RBD-ACE2-interaction on a biochemically basis, a cellular infection assay was employed to confirm neutralization potency. Therefore, human Caco-2 cells were co-incubated with icSARS-CoV-2-mNG and serial dilutions ranging from 1 µM to 1.6 nM of the inhibitory Nbs NM1226, NM1228, NM1224 and NM1230. Additionally, NM1223 was applied as representative of a non-neutralizing Nb according to the previously performed multiplex binding assay. As control, the unspecific GFP-Nb was included. 48 post-infection neutralization potency was determined via automated fluorescence-microscopy of fixed and nuclear-stained cells. As readout cell count and infection rate were analyzed. (Figure 13). Percent of neutralization upon treatment with corresponding Nb dilutions compared to an infected control were plotted and via sigmoidal inhibition curve fits the half-maximal infection stated as IC₅₀ value was determined.

Overall, a high correlation between the data obtained from the multiplexed binding assay and the virus neutralization assay was observed. Representatives of Nb-Set1, NM1226 and NM1228, showed the highest neutralization potency with IC₅₀ values of ∼ 10 nM and -23 nM, whereas NM1223 (Nb-Set3) could not reduce viral infectivity efficiently. To pronouncedly reduce viral infectivity and prevent mutational escape of the virus, it is inevitable to use combinations of Nbs targeting diverse epitopes. Therefore the most potent candidates derived from Nb-Set1 (NM1226, NM1228) and Nb-Set2 (NM1230) were combined and they were tested in the multiplexed binding assay and for viral neutralization.

The combination of Nbs NM1226 and NM1230 showed an increased effect in competing with RBD binding of ACE2 in the multiplexed binding assay as illustrated by a IC₅₀ of 0.42 nM which is 2- or 5-fold lower compared to individual NM1226 or NM1230, respectively (Figure 7 A). Notably, the IC₅₀ measured for the combination of Nbs NM1228 and NM1230 did not exceed the IC₅₀ identified for NM1228 alone indicating that NM1228 has a very high inhibiting effect. (Figure 7 A). When testing the Nb combinations in the viral neutralization assay, significantly improved effects for both of them were observed illustrated by an IC₅₀ of 4.09 nM for NM1226/NM1230 and 5.84 nM for NM1228/NM1230 (Figure 7 B, Figure 14). From these findings it is concluded, that a combinatorial treatment with two Nbs targeting different epitopes within the RBD: ACE2 interaction site is highly beneficial for viral neutralization.

### The NeutrobodyPlex - High-throughput detection of neutralizing antibodies in serum samples of patients after SARS-CoV-2 infection

To test the hypothesis that the RBD Nbs of the invention covering large parts of the RBD:ACE2 interaction site might be suitable tools to monitor the emergence and presence of neutralizing antibodies in patients, a high-throughput competitive binding assay was set up by combining the most potent neutralizing Nb combinations with a recently developed, automatable multiplex immunoassay (Becker, M., et al., "Going beyond clinical routine in SARS-CoV-2 antibody testing - A multiplex corona virus antibody test for the evaluation of cross-reactivity to endemic coronavirus antigens" medRxiv, (2020): p. 2020.07.17.20156000).

For the assay the previously generated color-coded beads comprising RBD, S1 domain or trimeric Spike were incubated with serum samples in addition to dilution series (0.08 nM - 1.26 µM) of the Nb combinations NM1226/NM1230 or NM1228/NM1230 and human IgGs bound to the respective antigens were detected. Depending on the Nb concentration, neutralizing antibodies targeting RBD:ACE2 interaction site within the serum samples are shifted to the unbound state resulting in a loss of the detectable signal (Figure 8A).

By analyzing IgGs from serum samples on RBD a distinct signal reduction in the presence of increasing Nb concentrations for all tested samples was detected (Figure 8B, Figure 15A), indicating that all patients comprise a substantial fraction of IgGs targeting the RBD:ACE2 interaction site. As expected, no changes of antibody signal was observed when analyzing the trimeric spike protein, indicating the presence of multiple IgGs binding to epitopes which are not within the RBD:ACE2 interaction site (Figure 8B, Figure 15A). To demonstrate the the approach allows a detailed resolution to determine the presence of IgGs targeting the RBD:ACE2 interaction site the effect of competing Nbs on two selected two selected serum samples was highlighted. For sample #289 a clear replacement of IgGs was observed when measuring antibody binding to RBD and S1 domain upon addition of competing Nbs while for sample #265 only a slight reduction of the IgG signal on S1 domain was detectable (Figure 8C, Figure 15B).

To compare the NeutrobodyPlex approach using RBD specific Nbs with conventional antibodies, the neutralizing mouse antibody MM43 was applied ((Gorshkov, K., et al., "Quantum Dot-Conjugated SARS-CoV-2 Spike Pseudo-Virions Enable Tracking of Angiotensin Converting Enzyme 2 Binding and Endocytosis"ACS Nano, (2020)) in a similar setting. Here, high cross-reactive signals were detected in all five serum samples (Figure 15C) from which it was concluded, that mouse antibodies blocking the RBD:ACE2 interaction site are not suitable in such a testing system as they bear the risk to be falsely detected by an anti-human IgG sensor. This underlines that due to their unique features, Nbs are highly suitable tools for such competitive binding analysis to detect neutralizing serum antibodies on a high-throughput basis.

In summary, the data presented herein reveal that the NeutrobodyPlex provide a suitable detection system to monitor the presence of RBD targeting antibodies in patient samples which can be doubtlessly considered to mediate a neutralizing and protective immune response.

Finally the NeutrobodyPlex was validated by analyzing 18 serum samples of convalescent SARS-CoV-2 patients and four control samples from healthy donors using one consistent Nb concentration (1.26 µM). Within the tested serum cohort all donors infected with SARS-CoV-2 showed a distinct quantity of neutralizing antibodies, most clearly visible by testing the samples on RBD-coupled beads (Figure 9). For the S1 domain, clear differences in signal reduction could be observed among the serum samples, indicating varying ratios of coinciding neutralizing and non-neutralizing IgGs. However, when IgG binding to the homotrimeric Spike protein was tested, upon addition of the competing Nbs, a strong signal from non-neutralizing IgGs was observed overlaying the loss of signal derived from neutralizing serum IgGs (Figure 9).

### Discussion

Here the identification of 1 novel RBD specific Nbs is described derived from an immunized animal (*Vicugna pacos*). According to their sequences these Nbs can be clustered in 8 unique families representing different germ lines which indicates a prominent immune response of the animal towards the fully glycosylated antigen used for immunization. All identified monovalent Nbs except NM1225 show high affinities in the low nanomolar range. Thus, these Nbs have not to be reformatted into bivalent formats e.g. by fusing to a Fc domain or by combining multiple binding sites as previously shown for other RBD targeting Nbs.

For functional analysis, a recently developed *in vitro* multiplex assay (Becker, M., et al., "Going beyond clinical routine in SARS-CoV-2 antibody testing - A multiplex corona virus antibody test for the evaluation of cross-reactivity to endemic coronavirus antigens" medRxiv, (2020): p. 2020.07.17.20156000) was employed to monitor binding of RBD, S1-domain and trimeric spike to ACE2. With this assay the simultaneous identification of eight inhibiting Nbs targeting those spike-derived proteins was possible. Interestingly, IC₅₀ values obtained for inhibitory Nbs on RBD and trimeric spike shows a higher correlation compared to the IC₅₀ obtained for the S1-domain. This suggests a functional folding of the RBD as exposed within the trimeric spike which is highly comparable to the folding of the isolated RBD used for immunization and selection of Nbs in this study.

Based on a detailed epitope binning and mapping, five different Nb-Sets were identified of which three comprises inhibitory Nbs which were shown to target different epitopes within the RBD:ACE2 interaction site. The potency to inhibit viral binding to its cellular receptor was confirmed a cellular viral neutralization assay using fully intact SARS-CoV-2 virus. By this it was noted that the measurable viral neutralization effect of the individual Nbs strongly correlates to data obtained from the in vitro screen. From that it can be concluded that the *in vitro* multiplex binding assay as presented here is highly relevant and suitable to identify binding molecules which are also functional to neutralize viral infection.

Based on these findings a novel diagnostic test was developed to monitor the presence and the emergence of neutralizing antibodies in serum samples of SARS-CoV-2 individuals. Using the most potent neutralizing Nb combinations of the invention, an distinct replacement of human IgGs was shown present in serum samples from binding to the RBD:ACE2 interaction site. Acknowledging previous studies those human IgGs are classified as neutralizing antibodies. Consequently, the approach presented herein using the well-defined Nbs for blocking the RBD:ACE2 interaction site in a multiplex binding assay shows for the first time an antigen-resolved analysis of the presence of human IgGs in reconvalescent individuals suffering from an SARS-CoV-2 infection.

In summary, the multiplex immunoassay as disclosed herein using competing Nbs provides a unique detection system to monitor the presence of neutralizing antibodies in patient samples. This novel and versatile high-throughput and automatable screening assay opens unique possibilities for a detailed classification of the individual immune status with regard to the development of protective antibodies and to monitor the efficiency of strongly needed vaccination campaigns.

## Claims

1. A nanobody directed against the SARS-CoV-2 (Severe acute respiratory syndrome coronavirus type 2) comprising:
a) the amino acid sequences (i) VAYGNMLRGYVVG (SEQ ID NO: 1) as CDR1, (ii) IDTSGEKKK (SEQ ID NO: 2) as CDR2 and (iii) NADAPWPPRPYSVIGTRTG (SEQ ID NO: 3) as CDR3; or
b) the amino acid sequences (i) VGSGFLFSGYAMN (SEQ ID NO: 4) as CDR1, (ii) ISNAGDITH (SEQ ID NO: 5) as CDR2 and (iii) HAPGVRVASGERND (SEQ ID NO: 6) as CDR3; or
c) the amino acid sequences (i) VGSGFTFSGYAIN (SEQ ID NO: 7) as CDR1, (ii) ISNAGDLTH (SEQ ID NO: 8) as CDR2 and (iii) HAPGVRVGTGERND (SEQ ID NO: 9) as CDR3 ; or
d) the amino acid sequences (i) SASGFAFSSVSMS (SEQ ID NO: 10) as CDR1, (ii) IDRDGGNGN (SEQ ID NO: 11) as CDR2 and (iii) RLGTRDHIMS (SEQ ID NO: 12) as CDR3; or
e) the amino acid sequences (i) ETSRSSLDAYAIG (SEQ ID NO: 13) as CDR1, (ii) ISSSSMRTE (SEQ ID NO: 14) as CDR2 and (iii) AAAGEYGRAWPGLDWYEFE (SEQ ID NO: 15) as CDR3; or
f) the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3; or
g) the amino acid sequences (i) ETSGRHFDIDDMG (SEQ ID NO: 19) as CDR1, (ii) ITTESSTT (SEQ ID NO: 20) as CDR2 and (iii) NAEMHPRSLDYALGNRD (SEQ ID NO: 21) as CDR3; or
h) the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3; or
i) the amino acid sequences (i) AASGRTHDWYTMG (SEQ ID NO: 25) as CDR1, (ii) INWSSGMTY (SEQ ID NO: 26) as CDR2 and (iii) NVHPFTSPD (SEQ ID NO: 27) as CDR3; or
j) the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3, or
k) amino acid sequences that have at least 90% sequence homology with the amino acid sequences as defined in a), b), c), d), e), f), g), h), i), or j).,
wherein the nanobody binds to the Receptor binding domain (RBD) of SARS-CoV-2, and wherein the nanobody, in its monovalent format, has a K_{D} of lower than 60 nM.

2. The nanobody of claim 1, comprising four framework regions (FR1 to FR4) and three complementarity determining regions (CDR1 to CDR3), the three complementarity determining regions consisting of one of the amino acid sequences a) to j), or of an amino acid sequence that has at least 90% sequence homology with one of the amino acid sequences a) to j).

3. The nanobody of claim 1 or 2, wherein the nanobody comprises an amino acid sequence selected from the group consisting of the amino acid sequences SEQ ID NO. 31, SEQ ID NO. 32, SEQ ID NO.33, SEQ ID NO. 34, SEQ ID NO. 35, SEQ ID NO. 36, SEQ ID NO. 37, SEQ ID NO. 38, SEQ ID NO. 39, SEQ ID NO. 40, or an amino acid sequence that has at least 90% sequence homology with one of the amino acid sequences SEQ ID NO. 31, SEQ ID NO. 32, SEQ ID NO.33, SEQ ID NO. 34, SEQ ID NO. 35, SEQ ID NO. 36, SEQ ID NO. 37, SEQ ID NO. 38, SEQ ID NO. 39, SEQ ID NO. 40.

4. The nanobody of any of claims 1 to 3, alone or in combination with at least one other nanobody as claimed in claims 1 to 3, for use as a drug.

5. The nanobody of any of claims 1 to 4 for use in the therapy or prophylaxis of a SARS-CoV-2-infection, wherein the nanobody is used alone or in combination with at least one another substance, which is selected from a therapeutically active substance and/or at least one another nanobody as claimed in any of claims 1 to 3.

6. The nanobody for use as claimed in claim 5, wherein, when used in combination, the nanobody and the at least one another substance, and/or nanobody, are used as two separate entities in the combination.

7. The nanobody for use as claimed in claim 5, wherein, when used in combination, the nanobody and the at least one another substance, and/or nanobody, are linked to each other, preferably directly or indirectly, preferably via a linker.

8. The nanobody for use as claimed in any of claims 4 to 7, wherein a nanobody comprising the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3 is used in combination with at least one another nanobody as claimed in claims 1 to 3.

9. The nanobody for use as claimed in any of claims 4 to 8, wherein a nanobody comprising the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3, or a nanobody comprising the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3 is used in the combination.

10. The nanobody of any of claims 1 to 3, alone or in combination with at least one other nanobody as claimed in claims 1 to 3, for use in diagnostic or prognostic methods.

11. The nanobody of any of claims 1 to 3 for use in *in vitro* methods for screening of the emergence and/or presence of neutralizing SARS-CoV-2 antibodies in a biological sample.

12. The nanobody of any of claims 1 to 3, alone or in combination with at least one other nanobody as claimed in claims 1 to 3, for use in determining the presence or absence of SARS-CoV-2 in a biological sample.

13. A pharmaceutical composition comprising at least one nanobody as defined in any of claims 1 to 3 in association with a pharmaceutically acceptable carrier.

14. The pharmaceutical composition of claim 11, wherein the composition comprises a nanobody comprising the amino acid sequences (i) VGSGFTFSGYAMN (SEQ ID NO: 31) as CDR1, (ii) ISNAGDLTH (SEQ ID NO: 32) as CDR2 and (iii) HAPGVRVGTGERKD (SEQ ID NO: 33) as CDR3 in combination with at least one other nanobody as claimed in claims 1 to 3.

15. The pharmaceutical composition of claim 12, wherein the pharmaceutical composition comprises a nanobody comprising the amino acid sequences (i) LGSGSLDYYAIG (SEQ ID NO: 19) as CDR1, (ii) IASSGDRTI (SEQ ID NO: 20) as CDR2 and (iii) AALQGSYYYTGFVANEYD (SEQ ID NO: 21) as CDR3, or a nanobody comprising the amino acid sequences (i) ESSGRHFDIDTMG (SEQ ID NO: 25) as CDR1, (ii) ITSEKSTV (SEQ ID NO: 26) as CDR2 and (iii) NAKMDPHSLDYALGNQV (SEQ ID NO: 27) as CDR3 in the combination.

16. A method for screening of the emergence and/or presence of SARS-CoV-2-neutralizing antibodies in a biological sample, **characterized in that** a nanobody as claimed in claims 1 to 3, alone or in combination with at least one other nanobody as claimed in claims 1 to 3 is used in the method.

17. A method for determining the presence or absence of SARS-CoV-2 in a biological sample, **characterized in that** a nanobody as claimed in claims 1 to 3, alone or in combination with at least one other nanobody as claimed in claims 1 to 3 is used in the method.

18. The method of claim 16 or 17, wherein a nanobody comprising the amino acid sequences (i) VGSGFTFSGYAMN (SEQ ID NO: 28) as CDR1, (ii) ISNAGDLTH (SEQ ID NO: 29) as CDR2 and (iii) HAPGVRVGTGERKD (SEQ ID NO: 30) as CDR3 is used in combination with at least one another nanobody as claimed in claims 1 to 3, preferably in combination with a nanobody comprising the amino acid sequences (i) LGSGSLDYYAIG (SEQ ID NO: 16) as CDR1, (ii) IASSGDRTI (SEQ ID NO: 17) as CDR2 and (iii) AALQGSYYYTGFVANEYD (SEQ ID NO: 18) as CDR3, or a nanobody comprising the amino acid sequences (i) ESSGRHFDIDTMG (SEQ ID NO: 22) as CDR1, (ii) ITSEKSTV (SEQ ID NO: 23) as CDR2 and (iii) NAKMDPHSLDYALGNQV (SEQ ID NO: 24) as CDR3.

19. The method as claimed in claim 14 or 15, **characterized in that** the biological sample is selected from the group consisting of a tissue sample, body fluid sample, preferably a sputum sample and saliva sample, blood sample, preferably a whole blood sample, plasma sample or serum sample.

20. A nucleic acid encoding an amino acid sequence comprising framework region 1, CDR1, framework region 2, CDR2, framework region 3, CDR3 and framework region 4, of the nanobody as claimed in any of claims 1 to 3.

21. A polypeptide comprising at least one nanobody as claimed in any of claims 1 to 9.


SEQUENCE LISTING

<110>  NMI Naturwissenschaftliches und Medizinisches Institut an
       der Universitaet Tuebingen

<120>  SARS-CoV-2-Nanobodies

<130>  3605P176EP

<160>  96

<170>  PatentIn version 3.5

<210>  1
<211>  13
<212>  PRT
<213>  Severe acute respiratory syndrome virus

<400>  1

Val Ala Tyr Gly Asn Met Leu Arg Gly Tyr Val Val Gly
1               5                   10

<210>  2
<211>  9
<212>  PRT
<213>  Severe acute respiratory syndrome virus

<400>  2

Ile Asp Thr Ser Gly Glu Lys Lys Lys
1               5

<210>  3
<211>  19
<212>  PRT
<213>  Severe acute respiratory syndrome virus

<400>  3

Asn Ala Asp Ala Pro Trp Pro Pro Arg Pro Tyr Ser Val Ile Gly Thr
1               5                   10                  15

Arg Thr Gly

<210>  4
<211>  13
<212>  PRT
<213>  Severe acute respiratory syndrome virus

<400>  4

Val Gly Ser Gly Phe Leu Phe Ser Gly Tyr Ala Met Asn
1               5                   10

<210>  5
<211>  9

<212>  PRT
<213>  Severe acute respiratory syndrome virus

<400>  5

Ile Ser Asn Ala Gly Asp Ile Thr His
1               5

<210>  6
<211>  14
<212>  PRT
<213>  Severe acute respiratory syndrome virus

<400>  6

His Ala Pro Gly Val Arg Val Ala Ser Gly Glu Arg Asn Asp
1               5                   10

<210>  7
<211>  13
<212>  PRT
<213>  Severe acute respiratory syndrome virus

<400>  7

Val Gly Ser Gly Phe Thr Phe Ser Gly Tyr Ala Ile Asn
1               5                   10

<210>  8
<211>  9
<212>  PRT
<213>  Severe acute respiratory syndrome virus

<400>  8

Ile Ser Asn Ala Gly Asp Leu Thr His
1               5

<210>  9
<211>  14
<212>  PRT
<213>  Severe acute respiratory syndrome virus

<400>  9

His Ala Pro Gly Val Arg Val Gly Thr Gly Glu Arg Asn Asp
1               5                   10

<210>  10
<211>  13
<212>  PRT
<213>  Severe acute respiratory syndrome virus

<400>  10

Ser Ala Ser Gly Phe Ala Phe Ser Ser Val Ser Met Ser
1               5                   10

<210>  11
<211>  9
<212>  PRT
<213>  Severe acute respiratory syndrome virus

<400>  11

Ile Asp Arg Asp Gly Gly Asn Gly Asn
1               5

<210>  12
<211>  10
<212>  PRT
<213>  Severe acute respiratory syndrome virus

<400>  12

Arg Leu Gly Thr Arg Asp His Ile Met Ser
1               5                   10

<210>  13
<211>  13
<212>  PRT
<213>  Severe acute respiratory syndrome virus

<400>  13

Glu Thr Ser Arg Ser Ser Leu Asp Ala Tyr Ala Ile Gly
1               5                   10

<210>  14
<211>  9
<212>  PRT
<213>  Severe acute respiratory syndrome virus

<400>  14

Ile Ser Ser Ser Ser Met Arg Thr Glu
1               5

<210>  15
<211>  19
<212>  PRT
<213>  Severe acute respiratory syndrome virus

<400>  15

Ala Ala Ala Gly Glu Tyr Gly Arg Ala Trp Pro Gly Leu Asp Trp Tyr
1               5                   10                  15

Glu Phe Glu

<210>  16
<211>  12
<212>  PRT

<213>  Severe acute respiratory syndrome virus

<400>  16

Leu Gly Ser Gly Ser Leu Asp Tyr Tyr Ala Ile Gly
1               5                   10


<210>  17
<211>  9
<212>  PRT
<213>  Severe acute respiratory syndrome virus

<400>  17

Ile Ala Ser Ser Gly Asp Arg Thr Ile
1               5


<210>  18
<211>  18
<212>  PRT
<213>  Severe acute respiratory syndrome virus

<400>  18

Ala Ala Leu Gln Gly Ser Tyr Tyr Tyr Thr Gly Phe Val Ala Asn Glu
1               5                   10                  15


Tyr Asp




<210>  19
<211>  13
<212>  PRT
<213>  Severe acute respiratory syndrome virus

<400>  19

Glu Thr Ser Gly Arg His Phe Asp Ile Asp Asp Met Gly
1               5                   10


<210>  20
<211>  8
<212>  PRT
<213>  Severe acute respiratory syndrome virus

<400>  20

Ile Thr Thr Glu Ser Ser Thr Thr
1               5


<210>  21
<211>  17
<212>  PRT
<213>  Severe acute respiratory syndrome virus

<400>  21

Asn Ala Glu Met His Pro Arg Ser Leu Asp Tyr Ala Leu Gly Asn Arg
1               5                   10                  15

Asp

<210>  22
<211>  13
<212>  PRT
<213>  Severe acute respiratory syndrome virus

<400>  22

Glu Ser Ser Gly Arg His Phe Asp Ile Asp Thr Met Gly
1               5                   10

<210>  23
<211>  8
<212>  PRT
<213>  Severe acute respiratory syndrome virus

<400>  23

Ile Thr Ser Glu Lys Ser Thr Val
1               5

<210>  24
<211>  17
<212>  PRT
<213>  Severe acute respiratory syndrome virus

<400>  24

Asn Ala Lys Met Asp Pro His Ser Leu Asp Tyr Ala Leu Gly Asn Gln
1               5                   10                  15

Val

<210>  25
<211>  13
<212>  PRT
<213>  Severe acute respiratory syndrome virus

<400>  25

Ala Ala Ser Gly Arg Thr His Asp Trp Tyr Thr Met Gly
1               5                   10

<210>  26
<211>  9
<212>  PRT
<213>  Severe acute respiratory syndrome virus

<400>  26

Ile Asn Trp Ser Ser Gly Met Thr Tyr
1               5

<210>  27
<211>  9
<212>  PRT
<213>  Severe acute respiratory syndrome virus

<400>  27

Asn Val His Pro Phe Thr Ser Pro Asp
1               5

<210>  28
<211>  13
<212>  PRT
<213>  Severe acute respiratory syndrome virus

<400>  28

Val Gly Ser Gly Phe Thr Phe Ser Gly Tyr Ala Met Asn
1               5                   10

<210>  29
<211>  9
<212>  PRT
<213>  Severe acute respiratory syndrome virus

<400>  29

Ile Ser Asn Ala Gly Asp Leu Thr His
1               5

<210>  30
<211>  14
<212>  PRT
<213>  Severe acute respiratory syndrome virus

<400>  30

His Ala Pro Gly Val Arg Val Gly Thr Gly Glu Arg Lys Asp
1               5                   10

<210>  31
<211>  127
<212>  PRT
<213>  Severe acute respiratory syndrome virus

<400>  31

Gln Val Gln Leu Val Glu Ser Gly Gly Gly Leu Val Gln Pro Gly Glu
1               5                   10                  15

Ser Leu Arg Leu Ser Cys Val Ala Tyr Gly Asn Met Leu Arg Gly Tyr
            20                  25                  30

Val Val Gly Trp Tyr Arg Gln Ala Pro Gly Lys Gln Arg Glu Leu Val
        35                  40                  45

Ala Gly Ile Asp Thr Ser Gly Glu Lys Lys Lys Tyr Ala Asp Ala Val
    50                  55                  60

Lys Gly Arg Phe Thr Ile Ser Arg Asp Asn Ala Gly Asn Thr Val Tyr
65                  70                  75                  80

Leu Gln Met Asn Ser Leu Lys Pro Glu Asp Thr Ala Val Tyr Tyr Cys
                85                  90                  95

Asn Ala Asp Ala Pro Trp Pro Pro Arg Pro Tyr Ser Val Ile Gly Thr
            100                 105                 110

Arg Thr Gly Tyr Trp Gly Arg Gly Ser Pro Val Thr Val Ser Ser
        115                 120                 125

<210>  32
<211>  122
<212>  PRT
<213>  Severe acute respiratory syndrome virus

<400>  32

Asp Val Gln Leu Val Glu Ser Gly Gly Gly Leu Val Gln Pro Gly Gly
1               5                   10                  15

Ser Leu Thr Leu Ser Cys Val Gly Ser Gly Phe Leu Phe Ser Gly Tyr
            20                  25                  30

Ala Met Asn Trp Tyr Arg Gln Ala Pro Gly Lys Ala Leu Glu Leu Val
        35                  40                  45

Ala Gly Ile Ser Asn Ala Gly Asp Ile Thr His Tyr Glu Glu Ala Met
    50                  55                  60

Lys Gly Arg Val Ala Ile Ser Arg Val Asn Asp Lys Asn Thr Val Tyr
65                  70                  75                  80

Leu Gln Met Asp Asp Leu Lys Pro Glu Asp Thr Ala Val Tyr Arg Cys
                85                  90                  95

His Ala Pro Gly Val Arg Val Ala Ser Gly Glu Arg Asn Asp Val Trp
            100                 105                 110

Gly Gln Gly Thr Gln Val Thr Val Ser Ser
        115                 120

<210>  33
<211>  122
<212>  PRT
<213>  Severe acute respiratory syndrome virus

<400>  33

Glu Val Gln Leu Val Glu Ser Gly Gly Gly Leu Val Arg Pro Gly Gly
1               5                   10                  15

Ser Leu Arg Leu Ser Cys Val Gly Ser Gly Phe Thr Phe Ser Gly Tyr
            20                  25                  30

Ala Ile Asn Trp Tyr Arg Gln Ala Pro Gly Lys Ala Leu Glu Leu Val
        35                  40                  45

Ala Gly Ile Ser Asn Ala Gly Asp Leu Thr His Tyr Glu Glu Ala Met
    50                  55                  60

Lys Gly Arg Val Ala Ile Ser Arg Ala Asn Asp Lys Asn Thr Val Tyr
65                  70                  75                  80

Leu Gln Met Asp Asp Leu Lys Pro Glu Asp Thr Ala Val Tyr Arg Cys
                85                  90                  95

His Ala Pro Gly Val Arg Val Gly Thr Gly Glu Arg Asn Asp Val Trp
            100                 105                 110

Gly Gln Gly Ala Gln Val Thr Val Ser Ser
        115                 120


<210>  34
<211>  118
<212>  PRT
<213>  Severe acute respiratory syndrome virus

<400>  34

Glu Val Gln Leu Val Glu Ser Gly Gly Gly Leu Val Gln Pro Gly Gly
1               5                   10                  15

Ser Leu Arg Leu Ser Cys Ser Ala Ser Gly Phe Ala Phe Ser Ser Val
            20                  25                  30

Ser Met Ser Trp Val Arg Leu Leu Pro Gly Lys Gly Thr Glu Trp Val
        35                  40                  45

Ala Glu Ile Asp Arg Asp Gly Gly Asn Gly Asn Tyr Glu Asp Ser Val
    50                  55                  60

Lys Gly Arg Phe Thr Ile Ser Arg Asp Asn Ala Lys Asn Thr Leu Phe

65                  70                  75                  80

Leu Gln Met Asn Ser Leu Val Pro Glu Asp Thr Ala Leu Tyr Tyr Cys
                85                  90                  95

Arg Leu Gly Thr Arg Asp His Ile Met Ser Gly Trp Gly Pro Gly Ala
            100                 105                 110

Pro Val Thr Val Ser Ser
        115

<210>  35
<211>  127
<212>  PRT
<213>  Severe acute respiratory syndrome virus

<400>  35

Asp Val Gln Leu Val Glu Ser Gly Gly Ser Leu Val Gln Pro Gly Gly
1               5                   10                  15

Ser Leu Arg Leu Ser Cys Glu Thr Ser Arg Ser Ser Leu Asp Ala Tyr
            20                  25                  30

Ala Ile Gly Trp Phe Arg Gln Ala Pro Gly Lys Glu Arg Glu Gly Val
        35                  40                  45

Ala Ser Ile Ser Ser Ser Ser Met Arg Thr Glu Tyr Ala Asp Ser Val
    50                  55                  60

Lys Gly Arg Phe Thr Ile Ser Arg Asp Asn Ala Lys Asn Thr Ala Tyr
65                  70                  75                  80

Leu Asp Met Asn Ser Leu Lys Pro Glu Asp Thr Ala Val Tyr Tyr Cys
                85                  90                  95

Ala Ala Ala Gly Glu Tyr Gly Arg Ala Trp Pro Gly Leu Asp Trp Tyr
            100                 105                 110

Glu Phe Glu Tyr Glu Gly Pro Gly Ser Pro Val Thr Val Ser Ser
        115                 120                 125

<210>  36
<211>  125
<212>  PRT
<213>  Severe acute respiratory syndrome virus

<400>  36

Gln Val Gln Leu Val Glu Ser Gly Gly Gly Ser Val Gln Pro Gly Gly
1               5                   10                  15

Ser Leu Arg Leu Ser Cys Leu Gly Ser Gly Ser Leu Asp Tyr Tyr Ala
            20                  25                  30

Ile Gly Trp Phe Arg Gln Ala Pro Gly Lys Glu Arg Glu Gly Val Ser
        35                  40                  45

Cys Ile Ala Ser Ser Gly Asp Arg Thr Ile Tyr Ala Asp Ser Val Lys
    50                  55                  60

Gly Arg Phe Thr Ile Ser Arg Asp Tyr Gly Lys Asn Thr Val Tyr Leu
65                  70                  75                  80

Gln Met Asn Ser Leu Lys Pro Glu Asp Thr Ala Met Tyr Tyr Cys Ala
                85                  90                  95

Ala Leu Gln Gly Ser Tyr Tyr Tyr Thr Gly Phe Val Ala Asn Glu Tyr
            100                 105                 110

Asp Tyr Trp Gly Gln Gly Ala Pro Val Thr Val Ser Ser
        115                 120                 125

<210>  37
<211>  124
<212>  PRT
<213>  Severe acute respiratory syndrome virus

<400>  37

Glu Val Gln Leu Val Glu Ser Gly Gly Gly Leu Val Gln Pro Gly Gly
1               5                   10                  15

Ser Leu Thr Leu Ser Cys Glu Thr Ser Gly Arg His Phe Asp Ile Asp
            20                  25                  30

Asp Met Gly Trp Tyr Arg Gln Ala Pro Gly Lys Gln Arg Glu Leu Val
        35                  40                  45

Ala Cys Ile Thr Thr Glu Ser Ser Thr Thr Tyr Ala Asp Ala Val Lys
    50                  55                  60

Gly Arg Phe Thr Ile Ser Arg Asp Asn Pro Asp Asn Thr Val Tyr Leu
65                  70                  75                  80

Gln Met Thr Asn Leu Lys Pro Glu Asp Thr Ala Val Tyr Tyr Cys Asn
                85                  90                  95

Ala Glu Met His Pro Arg Ser Leu Asp Tyr Ala Leu Gly Asn Arg Asp
            100                 105                 110

Tyr Trp Gly Gln Gly Ala Pro Val Thr Val Ser Ser
        115                 120

<210>  38
<211>  124
<212>  PRT
<213>  Severe acute respiratory syndrome virus

<400>  38

Asp Val Gln Leu Val Glu Ser Gly Gly Gly Leu Val Arg Pro Gly Gly
1               5                   10                  15

Ser Leu Arg Leu Ser Cys Glu Ser Ser Gly Arg His Phe Asp Ile Asp
            20                  25                  30

Thr Met Gly Trp Tyr Arg Gln Ala Pro Gly Lys Gln Arg Glu Leu Val
        35                  40                  45

Ala Ser Ile Thr Ser Glu Lys Ser Thr Val Tyr Ala Asp Ala Leu Lys
    50                  55                  60

Gly Arg Phe Thr Ile Ser Arg Asp Ile Pro Asp Asn Asn Val Tyr Leu
65                  70                  75                  80

Gln Met Asn Asn Leu Lys Pro Glu Asp Thr Ala Val Tyr Tyr Cys Asn
                85                  90                  95

Ala Lys Met Asp Pro His Ser Leu Asp Tyr Ala Leu Gly Asn Gln Val
            100                 105                 110

Phe Trp Gly Gln Gly Ser Arg Val Thr Val Ser Ser
        115                 120

<210>  39
<211>  117
<212>  PRT
<213>  Severe acute respiratory syndrome virus

<400>  39

Glu Val Gln Leu Gln Glu Ser Gly Gly Gly Leu Val Gln Pro Gly Glu
1               5                   10                  15

Ser Leu Arg Leu Ser Cys Ala Ala Ser Gly Arg Thr His Asp Trp Tyr
            20                  25                  30

Thr Met Gly Trp Phe Arg Gln Ala Pro Gly Lys Glu Arg Glu Phe Val
        35                  40                  45

Ala Arg Ile Asn Trp Ser Ser Gly Met Thr Tyr Tyr Ala Asp Ser Val
    50                  55                  60

Lys Gly Arg Phe Thr Ile Ser Arg Asp Asn Pro Lys Asn Thr Val Tyr
65                  70                  75                  80

Leu Gln Met Asn Ser Leu Thr Pro Asp Asp Thr Ala Val Tyr Tyr Cys
                85                  90                  95

Asn Val His Pro Phe Thr Ser Pro Asp Tyr Trp Gly Gln Gly Thr Arg
            100                 105                 110

Val Thr Val Ser Ser
        115

<210>  40
<211>  122
<212>  PRT
<213>  Severe acute respiratory syndrome virus

<400>  40

Gln Val Gln Leu Val Glu Ser Gly Gly Gly Leu Val Arg Pro Gly Gly
1               5                   10                  15

Ser Leu Arg Leu Ser Cys Val Gly Ser Gly Phe Thr Phe Ser Gly Tyr
            20                  25                  30

Ala Met Asn Trp Tyr Arg Gln Ala Pro Gly Lys Ala Leu Glu Leu Val
        35                  40                  45

Ala Gly Ile Ser Asn Ala Gly Asp Leu Thr His Tyr Glu Glu Pro Met
    50                  55                  60

Lys Gly Arg Val Ala Ile Ser Arg Ala Asn Asp Lys Asn Thr Val Tyr
65                  70                  75                  80

Leu Gln Met Asp Asp Leu Lys Pro Glu Asp Thr Ala Val Tyr Arg Cys
                85                  90                  95

His Ala Pro Gly Val Arg Val Gly Thr Gly Glu Arg Lys Asp Val Trp
            100                 105                 110

Gly Gln Gly Ala Gln Val Thr Val Ser Ser
        115                 120

<210>  41
<211>  22
<212>  PRT

<213>  Severe acute respiratory syndrome virus

<400>  41

Gln Val Gln Leu Val Glu Ser Gly Gly Gly Leu Val Gln Pro Gly Glu
1               5                   10                  15

Ser Leu Arg Leu Ser Cys
            20

<210>  42
<211>  15
<212>  PRT
<213>  Severe acute respiratory syndrome virus

<400>  42

Trp Tyr Arg Gln Ala Pro Gly Lys Gln Arg Glu Leu Val Ala Gly
1               5                   10                  15

<210>  43
<211>  37
<212>  PRT
<213>  Severe acute respiratory syndrome virus

<400>  43

Tyr Ala Asp Ala Val Lys Gly Arg Phe Thr Ile Ser Arg Asp Asn Ala
1               5                   10                  15

Gly Asn Thr Val Tyr Leu Gln Met Asn Ser Leu Lys Pro Glu Asp Thr
            20                  25                  30

Ala Val Tyr Tyr Cys
        35

<210>  44
<211>  12
<212>  PRT
<213>  Severe acute respiratory syndrome virus

<400>  44

Tyr Trp Gly Arg Gly Ser Pro Val Thr Val Ser Ser
1               5                   10

<210>  45
<211>  22
<212>  PRT
<213>  Severe acute respiratory syndrome virus

<400>  45

Asp Val Gln Leu Val Glu Ser Gly Gly Gly Leu Val Gln Pro Gly Gly
1               5                   10                  15

Ser Leu Thr Leu Ser Cys
            20

<210>  46
<211>  15
<212>  PRT
<213>  Severe acute respiratory syndrome virus

<400>  46

Trp Tyr Arg Gln Ala Pro Gly Lys Ala Leu Glu Leu Val Ala Gly
1               5                   10                  15

<210>  47
<211>  37
<212>  PRT
<213>  Severe acute respiratory syndrome virus

<400>  47

Tyr Glu Glu Ala Met Lys Gly Arg Val Ala Ile Ser Arg Val Asn Asp
1               5                   10                  15

Lys Asn Thr Val Tyr Leu Gln Met Asp Asp Leu Lys Pro Glu Asp Thr
            20                  25                  30

Ala Val Tyr Arg Cys
        35

<210>  48
<211>  12
<212>  PRT
<213>  Severe acute respiratory syndrome virus

<400>  48

Val Trp Gly Gln Gly Thr Gln Val Thr Val Ser Ser
1               5                   10

<210>  49
<211>  22
<212>  PRT
<213>  Severe acute respiratory syndrome virus

<400>  49

Glu Val Gln Leu Val Glu Ser Gly Gly Gly Leu Val Arg Pro Gly Gly
1               5                   10                  15

Ser Leu Arg Leu Ser Cys
            20

<210>  50
<211>  15

<212>  PRT
<213>  Severe acute respiratory syndrome virus

<400>  50

Trp Tyr Arg Gln Ala Pro Gly Lys Ala Leu Glu Leu Val Ala Gly
1               5                   10                  15


<210>  51
<211>  37
<212>  PRT
<213>  Severe acute respiratory syndrome virus

<400>  51

Tyr Glu Glu Ala Met Lys Gly Arg Val Ala Ile Ser Arg Ala Asn Asp
1               5                   10                  15


Lys Asn Thr Val Tyr Leu Gln Met Asp Asp Leu Lys Pro Glu Asp Thr
            20                  25                  30


Ala Val Tyr Arg Cys
        35


<210>  52
<211>  12
<212>  PRT
<213>  Severe acute respiratory syndrome virus

<400>  52

Val Trp Gly Gln Gly Ala Gln Val Thr Val Ser Ser
1               5                   10


<210>  53
<211>  22
<212>  PRT
<213>  Severe acute respiratory syndrome virus

<400>  53

Glu Val Gln Leu Val Glu Ser Gly Gly Gly Leu Val Gln Pro Gly Gly
1               5                   10                  15


Ser Leu Arg Leu Ser Cys
            20


<210>  54
<211>  15
<212>  PRT
<213>  Severe acute respiratory syndrome virus

<400>  54

Trp Val Arg Leu Leu Pro Gly Lys Gly Thr Glu Trp Val Ala Glu
1               5                   10                  15

<210>  55
<211>  37
<212>  PRT
<213>  Severe acute respiratory syndrome virus

<400>  55

Tyr Glu Asp Ser Val Lys Gly Arg Phe Thr Ile Ser Arg Asp Asn Ala
1               5                   10                  15

Lys Asn Thr Leu Phe Leu Gln Met Asn Ser Leu Val Pro Glu Asp Thr
            20                  25                  30

Ala Leu Tyr Tyr Cys
        35

<210>  56
<211>  12
<212>  PRT
<213>  Severe acute respiratory syndrome virus

<400>  56

Gly Trp Gly Pro Gly Ala Pro Val Thr Val Ser Ser
1               5                   10

<210>  57
<211>  22
<212>  PRT
<213>  Severe acute respiratory syndrome virus

<400>  57

Asp Val Gln Leu Val Glu Ser Gly Gly Ser Leu Val Gln Pro Gly Gly
1               5                   10                  15

Ser Leu Arg Leu Ser Cys
            20

<210>  58
<211>  15
<212>  PRT
<213>  Severe acute respiratory syndrome virus

<400>  58

Trp Phe Arg Gln Ala Pro Gly Lys Glu Arg Glu Gly Val Ala Ser
1               5                   10                  15

<210>  59
<211>  37
<212>  PRT
<213>  Severe acute respiratory syndrome virus

<400>  59

Tyr Ala Asp Ser Val Lys Gly Arg Phe Thr Ile Ser Arg Asp Asn Ala
1               5                   10                  15

Lys Asn Thr Ala Tyr Leu Asp Met Asn Ser Leu Lys Pro Glu Asp Thr
            20                  25                  30

Ala Val Tyr Tyr Cys
        35

<210>  60
<211>  12
<212>  PRT
<213>  Severe acute respiratory syndrome virus

<400>  60

Tyr Glu Gly Pro Gly Ser Pro Val Thr Val Ser Ser
1               5                   10

<210>  61
<211>  22
<212>  PRT
<213>  Severe acute respiratory syndrome virus

<400>  61

Gln Val Gln Leu Val Glu Ser Gly Gly Gly Ser Val Gln Pro Gly Gly
1               5                   10                  15

Ser Leu Arg Leu Ser Cys
            20

<210>  62
<211>  15
<212>  PRT
<213>  Severe acute respiratory syndrome virus

<400>  62

Trp Phe Arg Gln Ala Pro Gly Lys Glu Arg Glu Gly Val Ser Cys
1               5                   10                  15

<210>  63
<211>  37
<212>  PRT
<213>  Severe acute respiratory syndrome virus

<400>  63

Tyr Ala Asp Ser Val Lys Gly Arg Phe Thr Ile Ser Arg Asp Tyr Gly
1               5                   10                  15

Lys Asn Thr Val Tyr Leu Gln Met Asn Ser Leu Lys Pro Glu Asp Thr

20                  25                  30

Ala Met Tyr Tyr Cys
        35

<210>  64
<211>  12
<212>  PRT
<213>  Severe acute respiratory syndrome virus

<400>  64

Tyr Trp Gly Gln Gly Ala Pro Val Thr Val Ser Ser
1               5                   10

<210>  65
<211>  22
<212>  PRT
<213>  Severe acute respiratory syndrome virus

<400>  65

Glu Val Gln Leu Val Glu Ser Gly Gly Gly Leu Val Gln Pro Gly Gly
1               5                   10                  15

Ser Leu Thr Leu Ser Cys
            20

<210>  66
<211>  15
<212>  PRT
<213>  Severe acute respiratory syndrome virus

<400>  66

Trp Tyr Arg Gln Ala Pro Gly Lys Gln Arg Glu Leu Val Ala Cys
1               5                   10                  15

<210>  67
<211>  37
<212>  PRT
<213>  Severe acute respiratory syndrome virus

<400>  67

Tyr Ala Asp Ala Val Lys Gly Arg Phe Thr Ile Ser Arg Asp Asn Pro
1               5                   10                  15

Asp Asn Thr Val Tyr Leu Gln Met Thr Asn Leu Lys Pro Glu Asp Thr
            20                  25                  30

Ala Val Tyr Tyr Cys
        35

<210>  68
<211>  12
<212>  PRT
<213>  Severe acute respiratory syndrome virus

<400>  68

Tyr Trp Gly Gln Gly Ala Pro Val Thr Val Ser Ser
1               5                   10

<210>  69
<211>  22
<212>  PRT
<213>  Severe acute respiratory syndrome virus

<400>  69

Asp Val Gln Leu Val Glu Ser Gly Gly Gly Leu Val Arg Pro Gly Gly
1               5                   10                  15

Ser Leu Arg Leu Ser Cys
            20

<210>  70
<211>  15
<212>  PRT
<213>  Severe acute respiratory syndrome virus

<400>  70

Trp Tyr Arg Gln Ala Pro Gly Lys Gln Arg Glu Leu Val Ala Ser
1               5                   10                  15

<210>  71
<211>  37
<212>  PRT
<213>  Severe acute respiratory syndrome virus

<400>  71

Tyr Ala Asp Ala Leu Lys Gly Arg Phe Thr Ile Ser Arg Asp Ile Pro
1               5                   10                  15

Asp Asn Asn Val Tyr Leu Gln Met Asn Asn Leu Lys Pro Glu Asp Thr
            20                  25                  30

Ala Val Tyr Tyr Cys
        35

<210>  72
<211>  12
<212>  PRT
<213>  Severe acute respiratory syndrome virus

<400>  72

Phe Trp Gly Gln Gly Ser Arg Val Thr Val Ser Ser
1               5                   10

<210>  73
<211>  22
<212>  PRT
<213>  Severe acute respiratory syndrome virus

<400>  73

Glu Val Gln Leu Gln Glu Ser Gly Gly Gly Leu Val Gln Pro Gly Glu
1               5                   10                  15

Ser Leu Arg Leu Ser Cys
            20

<210>  74
<211>  15
<212>  PRT
<213>  Severe acute respiratory syndrome virus

<400>  74

Trp Phe Arg Gln Ala Pro Gly Lys Glu Arg Glu Phe Val Ala Arg
1               5                   10                  15

<210>  75
<211>  37
<212>  PRT
<213>  Severe acute respiratory syndrome virus

<400>  75

Tyr Ala Asp Ser Val Lys Gly Arg Phe Thr Ile Ser Arg Asp Asn Pro
1               5                   10                  15

Lys Asn Thr Val Tyr Leu Gln Met Asn Ser Leu Thr Pro Asp Asp Thr
            20                  25                  30

Ala Val Tyr Tyr Cys
        35

<210>  76
<211>  12
<212>  PRT
<213>  Severe acute respiratory syndrome virus

<400>  76

Tyr Trp Gly Gln Gly Thr Arg Val Thr Val Ser Ser
1               5                   10

<210>  77
<211>  22
<212>  PRT

<213>  Severe acute respiratory syndrome virus

<400>  77

Gln Val Gln Leu Val Glu Ser Gly Gly Gly Leu Val Arg Pro Gly Gly
1               5                   10                  15

Ser Leu Arg Leu Ser Cys
            20

<210>  78
<211>  15
<212>  PRT
<213>  Severe acute respiratory syndrome virus

<400>  78

Trp Tyr Arg Gln Ala Pro Gly Lys Ala Leu Glu Leu Val Ala Gly
1               5                   10                  15

<210>  79
<211>  37
<212>  PRT
<213>  Severe acute respiratory syndrome virus

<400>  79

Tyr Glu Glu Pro Met Lys Gly Arg Val Ala Ile Ser Arg Ala Asn Asp
1               5                   10                  15

Lys Asn Thr Val Tyr Leu Gln Met Asp Asp Leu Lys Pro Glu Asp Thr
            20                  25                  30

Ala Val Tyr Arg Cys
        35

<210>  80
<211>  12
<212>  PRT
<213>  Severe acute respiratory syndrome virus

<400>  80

Val Trp Gly Gln Gly Ala Gln Val Thr Val Ser Ser
1               5                   10

<210>  81
<211>  18
<212>  DNA
<213>  Artificial Sequence

<220>
<223>  Primer

<400>  81
cttctggcgt gtgaccgg                                                    18

<210>  82
<211>  53
<212>  DNA
<213>  Artificial Sequence

<220>
<223>  Primer

<400>  82
gttgcggccg cttagtggtg gtggtggtgg tgggggctgt ttgtctgtgt ctg          53


<210>  83
<211>  23
<212>  DNA
<213>  Artificial Sequence

<220>
<223>  Primer

<400>  83
gtcctggctg ctcttctaca agg                                           23


<210>  84
<211>  23
<212>  DNA
<213>  Artificial Sequence

<220>
<223>  Primer

<400>  84
ggtacgtgct gttgaactgt tcc                                           23


<210>  85
<211>  33
<212>  DNA
<213>  Artificial Sequence

<220>
<223>  Primer


<220>
<221>  misc_feature
<222>  (7)..(7)
<223>  n is a, c, g, or t

<220>
<221>  misc_feature
<222>  (10)..(10)
<223>  n is a, c, g, or t

<220>
<221>  misc_feature
<222>  (25)..(25)
<223>  n is a, c, g, or t

<220>

<221>  misc_feature
<222>  (28)..(28)
<223>  n is a, c, g, or t

<220>
<221>  misc_feature
<222>  (31)..(31)
<223>  n is a, c, g, or t

<400>  85
catggcnsan gtgcagctgg tggantcngg ngg                                    33


<210>  86
<211>  33
<212>  DNA
<213>  Artificial Sequence

<220>
<223>  Primer


<220>
<221>  misc_feature
<222>  (7)..(7)
<223>  n is a, c, g, or t

<220>
<221>  misc_feature
<222>  (10)..(10)
<223>  n is a, c, g, or t

<220>
<221>  misc_feature
<222>  (25)..(25)
<223>  n is a, c, g, or t

<220>
<221>  misc_feature
<222>  (28)..(28)
<223>  n is a, c, g, or t

<220>
<221>  misc_feature
<222>  (31)..(31)
<223>  n is a, c, g, or t

<400>  86
catggcnsan gtgcagctgc aggantcngg ngg                                    33


<210>  87
<211>  33
<212>  DNA
<213>  Artificial Sequence

<220>
<223>  Primer


<220>
<221>  misc_feature
<222>  (7)..(7)

<223>  n is a, c, g, or t

<220>
<221>  misc_feature
<222>  (10)..(10)
<223>  n is a, c, g, or t

<220>
<221>  misc_feature
<222>  (25)..(25)
<223>  n is a, c, g, or t

<220>
<221>  misc_feature
<222>  (31)..(31)
<223>  n is a, c, g, or t

<400>  87
catggcnsan gtgcagctgg tgganagygg ngg                                    33


<210>  88
<211>  33
<212>  DNA
<213>  Artificial Sequence

<220>
<223>  Primer


<220>
<221>  misc_feature
<222>  (7)..(7)
<223>  n is a, c, g, or t

<220>
<221>  misc_feature
<222>  (10)..(10)
<223>  n is a, c, g, or t

<220>
<221>  misc_feature
<222>  (25)..(25)
<223>  n is a, c, g, or t

<220>
<221>  misc_feature
<222>  (31)..(31)
<223>  n is a, c, g, or t

<400>  88
catggcnsan gtgcagctgc agganagygg ngg                                    33


<210>  89
<211>  38
<212>  DNA
<213>  Artificial Sequence

<220>
<223>  Primer

<220>
<221>  misc_feature
<222>  (22)..(22)
<223>  n is a, c, g, or t

<220>
<221>  misc_feature
<222>  (25)..(25)
<223>  n is a, c, g, or t

<400>  89
gtaggcccag ccggccatgg cnsangtgca gctggtgg                              38


<210>  90
<211>  39
<212>  DNA
<213>  Artificial Sequence

<220>
<223>  Primer



<220>
<221>  misc_feature
<222>  (22)..(22)
<223>  n is a, c, g, or t

<220>
<221>  misc_feature
<222>  (25)..(25)
<223>  n is a, c, g, or t

<400>  90
gtaggcccag ccggccatgg cnsangtgca gctgcagga                             39


<210>  91
<211>  35
<212>  DNA
<213>  Artificial Sequence

<220>
<223>  Primer



<220>
<221>  misc_feature
<222>  (12)..(12)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature

<222>  (28)..(28)
<223>  n is a, c, g, or t

<220>
<221>  misc_feature
<222>  (30)..(30)
<223>  n is a, c, g, or t

<220>
<221>  misc_feature
<222>  (33)..(33)
<223>  n is a, c, g, or t

<400>  91
gatgcggccg cnganganac ggtgaccngn ryncc                                35


<210>  92
<211>  35
<212>  DNA
<213>  Artificial Sequence

<220>
<223>  Primer



<220>
<221>  misc_feature
<222>  (12)..(12)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (28)..(28)
<223>  n is a, c, g, or t

<220>
<221>  misc_feature
<222>  (30)..(30)
<223>  n is a, c, g, or t

<220>
<221>  misc_feature
<222>  (33)..(33)
<223>  n is a, c, g, or t

<400>  92
gatgcggccg cnganganac ggtgaccngn gancc                                35


<210>  93
<211>  35
<212>  DNA

<213>  Artificial Sequence

<220>
<223>  Primer



<220>
<221>  misc_feature
<222>  (12)..(12)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (28)..(28)
<223>  n is a, c, g, or t

<220>
<221>  misc_feature
<222>  (33)..(33)
<223>  n is a, c, g, or t

<400>  93
gatgcggccg cnganganac ggtgaccngr ctncc                                35


<210>  94
<211>  35
<212>  DNA
<213>  Artificial Sequence

<220>
<223>  Primer



<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (28)..(28)
<223>  n is a, c, g, or t

<220>
<221>  misc_feature
<222>  (30)..(30)
<223>  n is a, c, g, or t

<220>
<221>  misc_feature
<222>  (33)..(33)
<223>  n is a, c, g, or t

<400>  94
gatgcggccg crctnganac ggtgaccngn ryncc                                35


<210>  95
<211>  35
<212>  DNA
<213>  Artificial Sequence

<220>
<223>  Primer



<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (28)..(28)
<223>  n is a, c, g, or t

<220>
<221>  misc_feature
<222>  (30)..(30)
<223>  n is a, c, g, or t

<220>
<221>  misc_feature
<222>  (33)..(33)
<223>  n is a, c, g, or t

<400>  95
gatgcggccg crctnganac ggtgaccngn gancc                                35


<210>  96
<211>  35
<212>  DNA
<213>  Artificial Sequence

<220>
<223>  Primer



<220>
<221>  misc_feature
<222>  (15)..(15)
<223>  n is a, c, g, or t

<220>

<221>  misc_feature
<222>  (18)..(18)
<223>  n is a, c, g, or t

<220>
<221>  misc_feature
<222>  (28)..(28)
<223>  n is a, c, g, or t

<220>
<221>  misc_feature
<222>  (33)..(33)
<223>  n is a, c, g, or t

<400>  96
gatgcggccg crctnganac ggtgaccngr ctncc                                35